# EUROPEAN PATENT SPECIFICATION

(11) **EP 3 132 024 B1**
(45) Date of publication and mention of the grant of the patent: **05.06.2024**
(21) Application number: 15780471.7
(22) Date of filing: 15.04.2015
(51) Int. Cl.: C12N 5/074, C12N 5/077, A61K 35/28, A61K 31/42, A61K 45/06, A61K 35/34, A61L 27/38, A61P 9/04, A61P 9/00

(54) **CHEMICALLY INDUCED PLURIPOTENT STEM CELLS FOR SAFE THERAPEUTIC APPLICATIONS**
CHEMISCH INDUZIERTE, PLURIPOTENTE STAMMZELLEN FÜR SICHERE THERAPEUTISCHE ANWENDUNGEN
CELLULES SOUCHES PLURIPOTENTES INDUITES CHIMIQUEMENT POUR APPLICATIONS THÉRAPEUTIQUES SÛRES

(30) Priority: 17.04.2014 US 201414255789
(43) Date of publication of application: 22.02.2017
(73) Proprietor: IPS Heart Inc., Sugar Land, TX 77478 (US)
(72) Inventor: ASHRAF, Muhammad, Cincinnati, Ohio 45249 (US)
(74) Representative: Wittop Koning, Tom Hugo
(86) International application number: PCT/US2015/026016
(87) International publication number: WO 2015/160982

(56) References cited:
- WO-A1-2013/024410
- US-A1- 2009 076 103
- US-A1- 2010 184 051
- US-A1- 2011 014 164
- US-A1- 2011 201 110
- US-A1- 2012 197 189
- US-A1- 2012 301 438
- US-A1- 2013 035 374
- US-A1- 2013 178 506
- US-A1- 2013 189 781
- MIN XIE ET AL: "Small Molecules for Cell Reprogramming and Heart Repair: Progress and Perspective", ACS CHEMICAL BIOLOGY, vol. 9, no. 1, 17 January 2014 (2014-01-17), pages 34-44, XP055364113, US ISSN: 1554-8929, DOI: 10.1021/cb400865w
- RUSSE LL ET AL.: 'Targeting native adult heart progenitors with cardiogenic smallmolecules' ACS CHEMICAL BIOLOGY vol. 7, no. ISS. 6, 13 April 2012, pages 1067 - 1076, XP055359501
- TERRI T. NI ET AL: "Discovering Small Molecules that Promote Cardiomyocyte Generation by Modulating Wnt Signaling", CHEMISTRY AND BIOLOGY., vol. 18, no. 12, 1 December 2011 (2011-12-01), pages 1658-1668, XP055552745, GB ISSN: 1074-5521, DOI: 10.1016/j.chembiol.2011.09.015

## Description

### BACKGROUND

Induced pluripotent stem (iPS) cells are important source for progenitors, such as cardiac progenitors, for drug discovery and the treatment of disease, e.g., infarcted myocardium. Due to inherent properties of iPS cells to form teratomas, it becomes very important to generate iPS cells without producing tumors for use in clinics. Given the importance of these concerns, efforts have been made to improve the reprogramming efficiency and several methods have been devised for the non-viral generation of IPS cells. Various growth factors and chemical compounds, such as DNA methyltransferase inhibitor (5'-azacytidine and RG108), histone deacetylase inhibitors (e.g., valproic acid), histone methyltransferase inhibitor (BIX-01294), Wnt3A, and ALK5 inhibitor, have been found to improve the induction efficiency (1-6) With different experimental manipulations, iPS cells can be induced to cardiac lineage cells prior to their transplantation. These procedures are labor intense and not efficient in producing unlimited number of cells for the treatment of CVS diseases. Thus a need exists for methods to produce safe iPS cells for the use in the clinic and for drug development. This invention satisfies this need and provides related advantages as well.

### SUMMARY OF THE DISCLOSURE

The invention is set out in the appended set of claims, and describes a chemically modified induced pluripotent stem (iPS) cell characterized by DNA hypomethylation. According to the invention, the cell is modified by contacting the cell with an effective amount of about 0.5 µM to about 30 µM of an isoxazole or isoxazole similar compound. The iPS cell can be derived from a parent cell selected from the group consisting of a bone marrow cell, a myoblast, a cord blood cell, a small juvenile stem cell, an adult peripheral blood cell, a mononuclear cell, or a skin fibroblast. The cardiac cell preferably overexpresses one or more cardiac genes or markers, non-limiting examples of such include Nkx 2.5, GATA4, αMHC, Sarcomeric actin, Gαi, mir-133, mir-762, CCL7, CXCR2, CXC5, integral membrane protein 2A, and ephrin A3. The cardiac cell preferably under expresses one or more pluripotent genes or markers, non-limiting examples of such include one or more of miR-290-295 cluster, let-7 family, Max and/or under expresses one or more DNA methyltransferase genes Dnmt1, Dnmt3b.

In one aspect, the chemically modified iPS cell was created by a method comprising contacting a parent cell with an effective amount of a DNA methyltransferase inhibitor to upregulate Oct4. Non-limiting examples of DNA methyltransferease inhibitors include 5'-azacytidine, 5-aza-2'-deoxycytidine, MG98, zebularine and RG108.The iPS cell was preferably created by a method that excludes the insertion of exogenous genes into the parent cell.

The parent cells can e.g. be cardiac progenitors that were preconditioned with electrical stimulation. The parent cells can e.g. be stem cells that express Sca 1, pluripotency and cardiac genes as described herein.

Further provided by this disclosure is a population of cells having the characteristics as described above. In one aspect, the population is substantially homogenous or clonal.

Non-limiting examples of stem cells are selected from a bone marrow cell, a myoblast, a cord blood, an electrically stimulated cardiac progenitor, a SJSC, an adult peripheral blood, a mononuclear cell, and a skin fibroblast cell. The methods provided herein can produce a cardiac lineage cell that overexpresses one or more cardiac genes or markers. Non-limiting examples of the one or more cardiac gene or marker are Nkx 2.5, GATA4, αMHC, Sarcomeric actin, Gαi, mir-133, mir-762, CCL7, CXCR2, CXC5, integral membrane protein 2A and ephrin A3. In addition or alternatively, the cardiac lineage can under express one or more pluripotent genes or markers. Non-limiting examples of the one or more pluripotency gene or marker are one or more of miR-290-295 cluster, let-7 family and/or expresses one or more DNA methyltransferase genes, Dnmt1, Dnmt3b, and Max.

The stem cell is an iPS cell. In one aspect, the method to prepare the iPSC comprises contacting a parent cell with an effective amount of a DNA methyltransferase inhibitor to upregulate Oct4. Non-limiting examples of DNA methyltransferease inhibitors include 5'- azacytidine, 5-aza-2'-deoxycytidine, MG98, zebularine and RG108. In one aspect, the iPS cell was created by a method that excludes the insertion of exogenous genes into the parent cell.

Further described herein are methods to prepare populations of cells, such as substantially homogenous or clonal population of cells, as described herein. They can be further modified by comprising, or alternatively consisting essentially of, or yet further consisting or a detectable label, by inserting into the cell the detectable label.

Yet further described is a method for regenerating cardiac muscle tissue or to promote the replacement of cardiac scar tissue in a patient in need thereof, by administering to the patient one or more of an effective amount of isoxazole, an isoxazole similar compound and/or an effective amount of the isolated cell as described herein.

Yet further described is a method for treating cardiac disease in a patient in need thereof, by administering to the patient an effective amount of isoxazole, an isoxazole similar compound and/or an effective amount of the isolated cell or the population of cells as described herein.

Patients treated by the disclosed methods include a mammalian patient. Non-limiting examples of such include a murine, an equine, a bovine, a feline, a canine or a human patient. Cells may be autologous or allogeneic to the patient.

Yet further described are kits to prepare the cells or administer the therapies as described herein, including the compositions or agents and instructions for use.

### BRIEF DESCRIPTION OF THE FIGURES

The patent or application file contains at least one drawing executed in color. Copies of this patent or patent application publication with color drawing(s) will be provided by the Office upon request and payment of necessary fee.

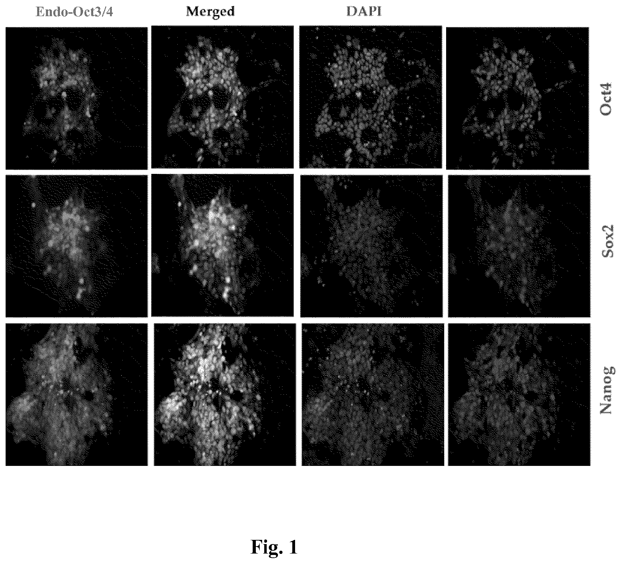

**Fig. 1** shows characterization of IPS cells for the expression of pluripotency markers. Representative photomicrographs showing IPS clones expressing embryonic stem cells (ESC) specific markers Oct3/4, Sox2, Nanog (red fluorescence) and endogenous Oct4 (green fluorescence). Nuclei were stained with DAPI (blue fluorescence).

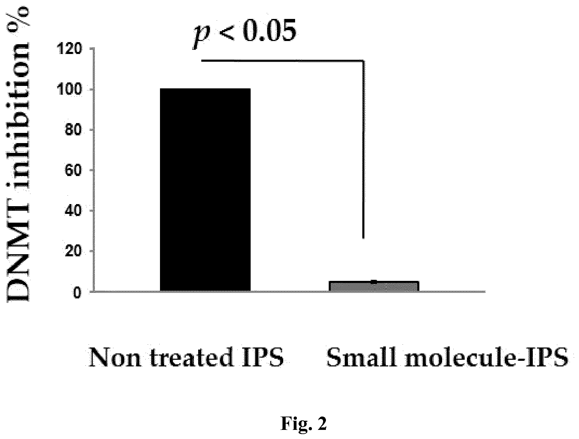

**Fig. 2** shows the results of DNA methyltransferase (DNMT) activity assay. DNA methylation analysis showing significant (95%) inhibition of DNA methyltransferase activity in small molecule treated IPS cells in comparison to the nontreated IPS cells.

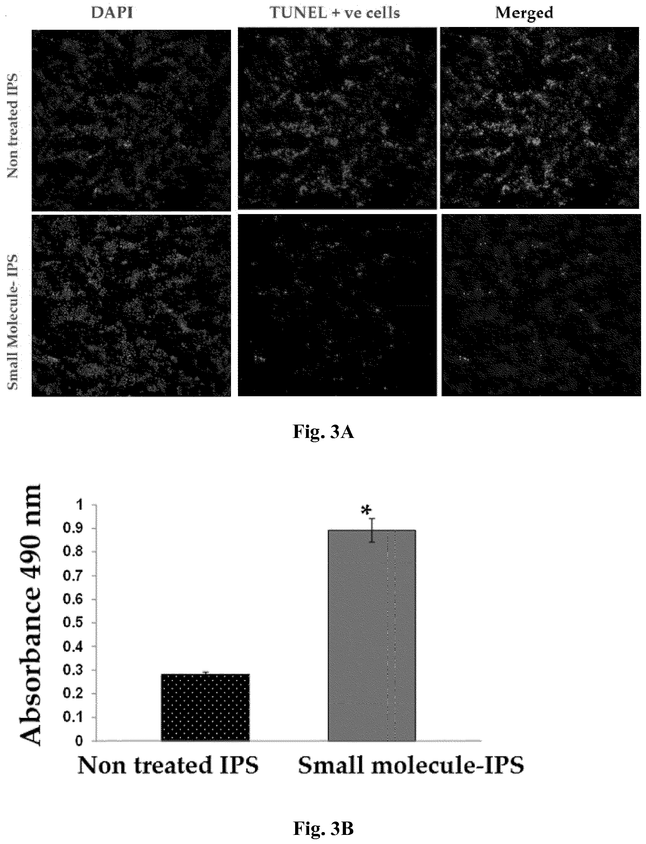

**Figs. 3A and 3B** show that small molecule treatment induces cytoprotection *in vitro* and that small molecule treatment prevents oxidant induced apoptosis and increases the proliferation. In **Fig. 3A** apoptosis was determined by TUNEL assay. Fewer TUNEL positive cells/ microscopic field were observed in small molecule pretreated IPS as compared to control non-treated IPS cells. In **Fig. 3B** an increase in mitogenic response of small molecule treated IPS was significantly noted in comparison with non-treated IPS cells. All values were expressed as mean ± SEM, ** p*< 0.05 *vs.* control.

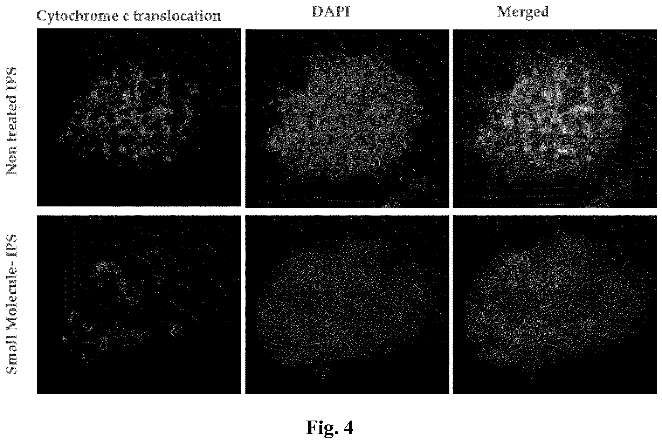

**Fig. 4** shows Cytochrome c translocation to cytoplasm (a sign of cell injury). Immunostaining of cytochrome c(red) in non-treated and small molecule treated IPS cells after exposure to H₂O₂ (100 µmol),(merged images with DAPI), (original magnifications; 200x). Small molecule treated IPS cells show a significant decrease in cytochrome c translocation to cytoplasm as compared to the non-treated IPS cells.

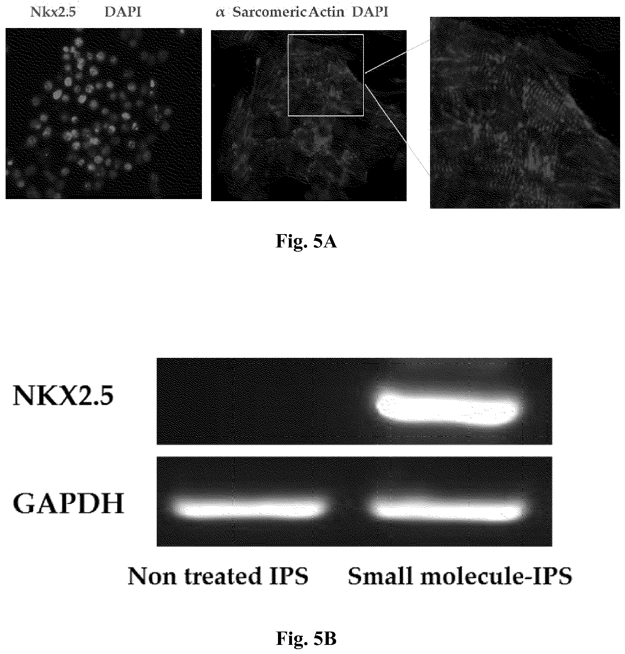

**Figs. 5A and 5B** show small molecule mediated cardiac differentiation of IPS cells *in vitro.* **Fig. 5A** shows immunostaining of cardiac specific gene NKx2.5 and α Sarcomeric actin in small molecule treated IPS cells (merged images with DAPI), (original magnifications; 400x). **Fig. 5B** is RT-PCR analysis of cardiomyocyte specific marker, Nkx2.5, in small molecule treated IPS cells in comparison with non-treated IPS cells. Small molecule treated IPS cells shows significant upregulation of NKx2.5 as compared to non-treated IPS cells.

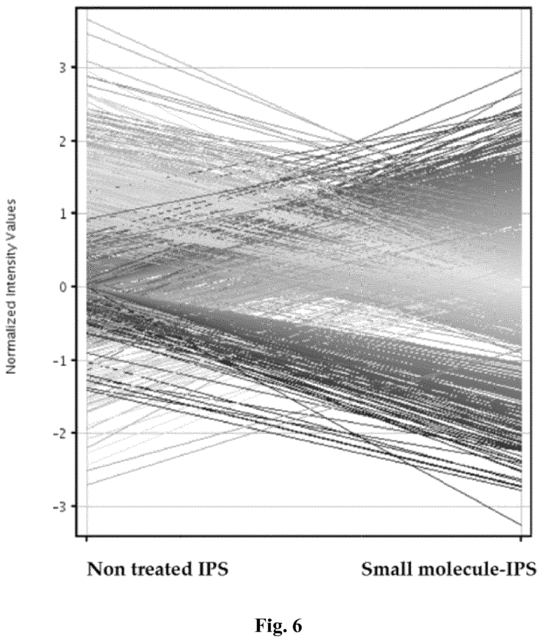

**Fig. 6** shows small molecule treatment induces Global DNA hypomethylation by targeting G-protein coupled receptor (GPCR) signaling.

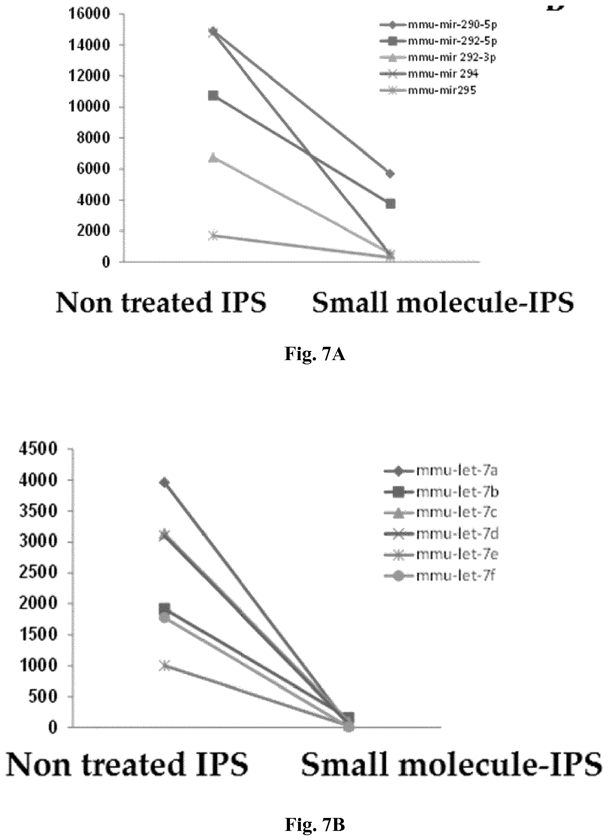

**Figs. 7A-7D** show miR- Microarray analysis of small molecule treated IPS vs non-treated IPS cells. Microarray analysis showing miR expression profile in non-treated IPS cells was quite different from small molecule treated IPS cells. **(****Figs. 7A-7D****)** Critical miRs known for reprogramming and differentiation as observed in non-treated and small molecule treated IPS cells. miR Microarray analysis showed upregulation of cardiac specific mir -133, mir-762 and down regulation of pluripotency associated miR-290-295 cluster and let-7 family in small molecule treated IPS cells.

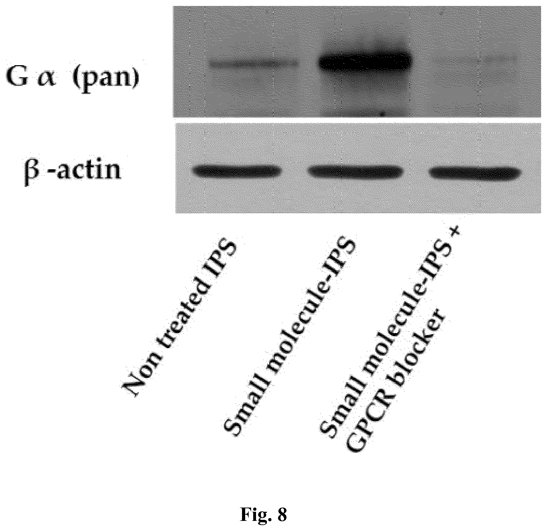

**Fig. 8** shows GPCR signaling in Small molecule treated SIPs cells. Western blot analysis shows significant upregulation of Gα (pan) in small moecule treated IPS cells as compared to the non-treated IPS cells which was blocked by the concomitant use of GPCR blocker, pertussis toxin.

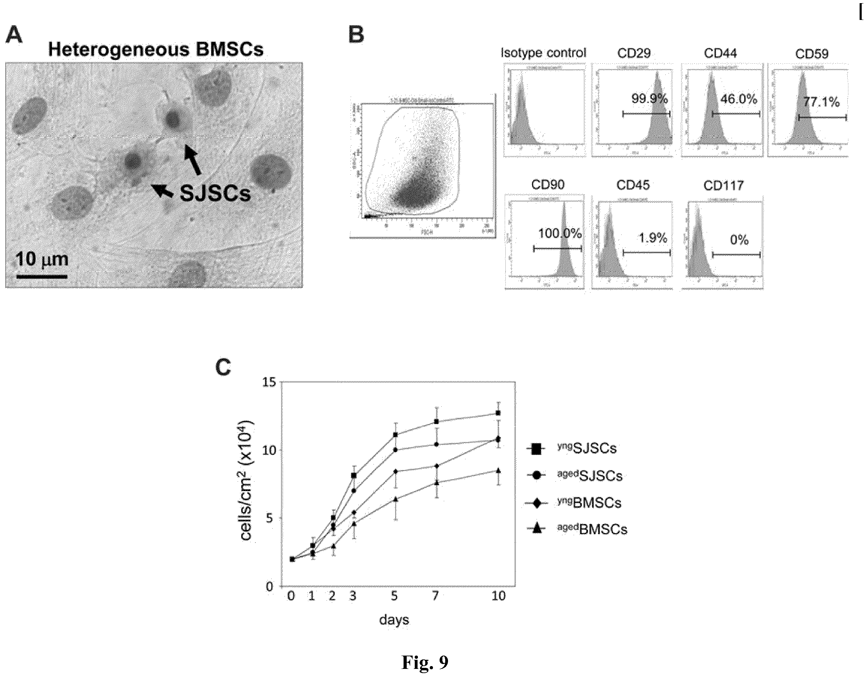

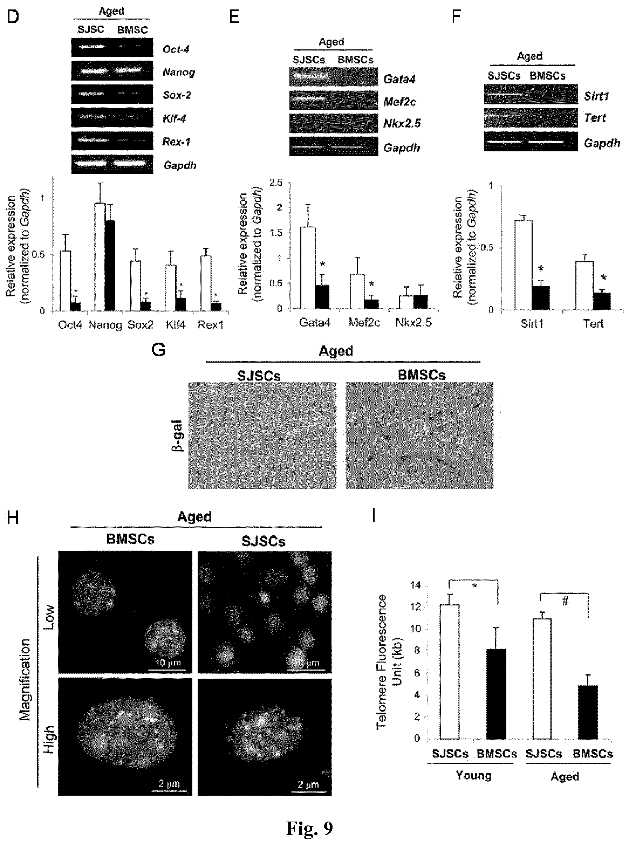

**Figs. 9A-9I** show the characterization of small juvenile stem cells (SJSCs) from heterogeneous bone marrow-derived stem cells (BMSCs) from young and old mouse described in Experiment No. 2 **Figs. 9A and 9B** show that both aged and young SJSCs were positive for CD29, CD44,CD59, and CD90 but negative for CD45 and CD117 B. In **Fig. 9C****,** cell growth curves show that the cell proliferation rate was higher in SJSCs than in BMSCs. Compared with aged BMSCs, aged SJSCs showed higher expression of pluripotency markers such as octamer-binding transcription factor 4 (Oct-4), Nanog, sex-determining region Y box 2 (Sox-2), Kruppel-like factor 4 (Klf-4), and Rex-1. **Fig. 9D** shows that cardiogenic differentiation markers such as Gata-4 and myocyte-specific enhancer factor 2C (Mef2c). **Fig. 9E** shows that antiaging markers such as sirtuin 1 (Sirt1) and telomerase reverse transcriptase (Tert) are expressed. In **Fig. 9F** marker expression as examined by RT-PCR. In **Fig. 9G****,** SJSCs from aged bone marrow showed less senescence-associated β-galactosidase (β-gal) expression compared with other cells in aged BM. **Fig. 9H** is confocal microscopy showing that aged SJSCs had a higher density of telomeres compared with aged BMSCs. **Fig. 9I** is auantitative fluorescence in situ hybridization (FISH) analysis demonstrated that SJSCs maintain longer telomeres compared with BMSCs. Telomere shortening was delayed in aged SJSCs compared with aged BMSCs.

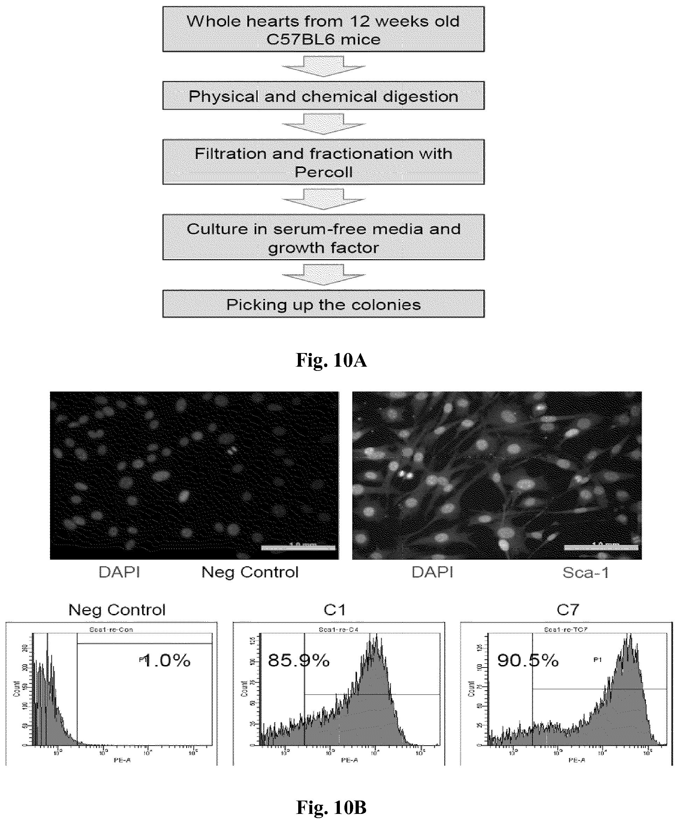

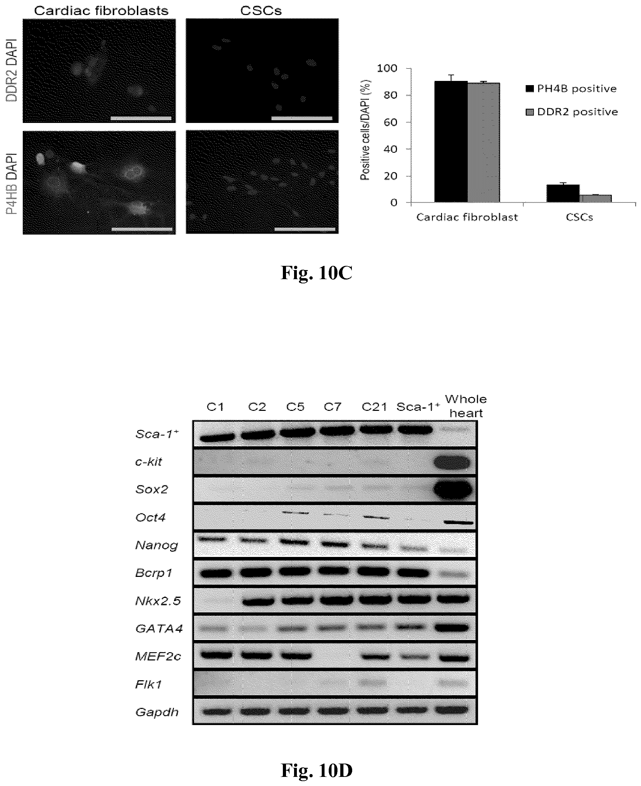

**Figs. 10A-10D** show characterization of Sca-1⁺ CSCs. **Fig. 10A** is a brief description of the procedure of CSCs isolation. **Fig. 10B** shows Sca-1⁺ expression in isolated CSCs was validated by immunocytochemistry and flowcytometry. (red=Sca-1⁺; blue=DAPI). In **Fig. 10C** Discoidin domain receptor 2 (DDR2) and prolyl-4-hydroxylase beta (P4HB) antibodies were rarely positive in CSC cultures. **Fig. 10D** shows that expression of a hematopoietic progenitor marker (c-kit), pluripotency markers (Oct-4, Sox2, Nanog), a stem cell side population marker (Bcrp1), early cardiac lineage markers (Nkx2.5, GATA4, MEF2C), and a vascular progenitor marker (Flk1) in isolated Sca-1⁺ cell colonies as analyzed by RT-PCR. "C" stands for cultured colonies and their identity numbers. They maintained about 80~90% of Sca-1⁺ positive cells from passage 5 to 30, measured by flowcytometry. All experiments were performed within this passage. Bar=100 µm.

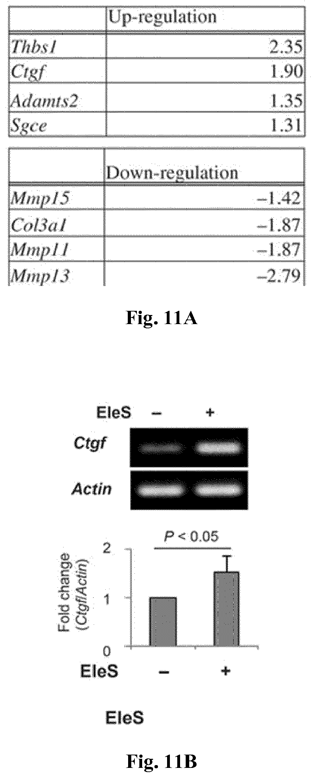

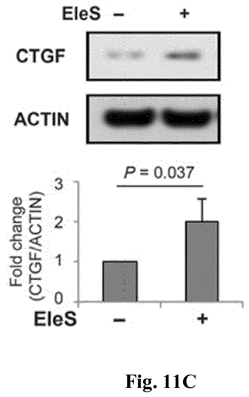

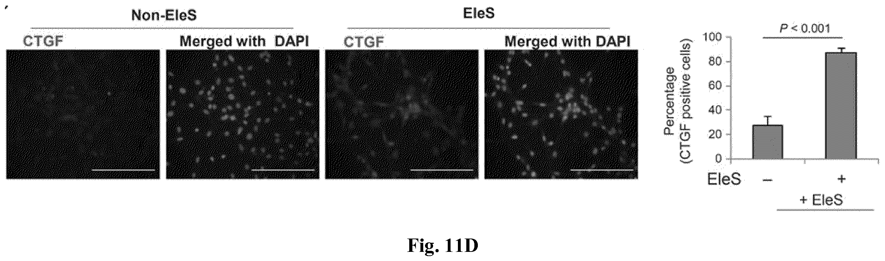

**Figs. 11A-11D** show that CTGF is a major downstream factor for EleS-induced FAK/AKT pathways. CTGF is a major downstream factor for EleS-induced FAK/AKT pathways. **Fig. 11A** shows real-time PCR-based gene expression profiling was performed for ECM and cell adhesion molecules. Four of each up-regulated and down-regulated genes were selected based on fold change. In **Figs. 11B** **and** **11C** mRNA expression of Ctgf was confirmed by conventional RT-PCR and real-time PCR (n = 5). **Fig. 11D** shows CTGF positivity by immunocytochemistry was higher in EleSCSCs when compared with Non-EleSCSCs.

### DETAILED DESCRIPTION

### Definitions

The practice of the present invention will employ, unless otherwise indicated, conventional techniques of tissue culture, immunology, molecular biology, microbiology, cell biology and recombinant DNA, which are within the skill of the art. See, e.g., Sambrook, Fritsch and Maniatis (1989) Molecular Cloning: A Laboratory Manual, 2nd edition; F. M. Ausubel, et al. eds. (1987) Current Protocols In Molecular Biology; the series Methods in Enzymology (Academic Press, Inc.): PCR 2: A Practical Approach (1995) (M.J. MacPherson, B.D. Hames and G.R. Taylor eds.); Harlow and Lane, eds. (1988) Antibodies, A Laboratory Manual; Harlow and Lane, eds. (1999) Using Antibodies, A Laboratory Manual; and R.I. Freshney, ed. (1987) Animal Cell Culture.

All numerical designations, e.g., pH, temperature, time, concentration, and molecular weight, including ranges, are approximations which are varied ( + ) or ( - ) by increments of 1.0 or 0.1, as appropriate. It is to be understood, although not always explicitly stated that all numerical designations are preceded by the term "about". It also is to be understood, although not always explicitly stated, that the reagents described herein are merely exemplary and that equivalents of such are known in the art.

As used in the specification and claims, the singular form "a," "an" and "the" include plural references unless the context clearly dictates otherwise.

The terms autologous transfer, autologous transplantation, autograft and the like refer to treatments wherein the cell donor is also the recipient of the cell replacement therapy. The terms allogeneic transfer, allogeneic transplantation, allograft and the like refer to treatments wherein the cell donor is of the same species as the recipient of the cell replacement therapy, but is not the same individual. A cell transfer in which the donor's cells and have been histocompatibly matched with a recipient is sometimes referred to as a syngeneic transfer. The terms xenogeneic transfer, xenogeneic transplantation, xenograft and the like refer to treatments wherein the cell donor is of a different species than the recipient of the cell replacement therapy.

A "biocompatible scaffold" refers to a scaffold or matrix for tissue-engineering purposes with the ability to perform as a substrate that will support the appropriate cellular activity to generate the desired tissue, including the facilitation of molecular and mechanical signaling systems, without eliciting any undesirable effect in those cells or inducing any undesirable local or systemic responses in the eventual host. In other embodiments, a biocompatible scaffold is a precursor to an implantable device which has the ability to perform its intended function, with the desired degree of incorporation in the host, without eliciting an undesirable local or systemic effects in the host. Biocompatible scaffolds are described in U.S. Patent No. 6,638,369.

As used herein, a "cardiac patch" is a bioengineered 2D or 3-dimensional (3D) tissue patch comprising or containing iPS cells.

A "cardiomyocyte" or "cardiac myocyte" is a specialized muscle cell which primarily forms the myocardium of the heart. Cardiomyocytes have five major components: 1. cell membrane (sarcolemma) and T-tubules, for impulse conduction, 2. sarcoplasmic reticulum, a calcium reservoir needed for contraction, 3. contractile elements, 4. mitochondria, and 5. a nucleus. Cardiomyocytes can be subdivided into subtypes including, but not limited to, atrial cardiomyocyte, ventricular cardiomyocyte, SA nodal cardiomyocyte, peripheral SA nodal cardiomyocyte, or central SA nodal cardiomyocyte. Stem cells can be propagated to mimic the physiological functions of cardiomyocytes or alternatively, differentiate into cardiomyocytes. This differentiation can be detected by the use of markers selected from, but not limited to, myosin heavy chain, myosin light chain, actinin, troponin, tropomyosin, GATA4, Mef2c, and Nkx2.5.

The cardiomyocyte marker "myosin heavy chain" and "myosin light chain" are part of a large family of motor proteins found in muscle cells responsible for producing contractile force. These proteins have been sequenced and characterized, see for example GenBank Accession Nos. AAD29948, CAC70714, CAC70712, CAA29119, P12883, NP_000248, P13533, CAA37068, ABR18779, AAA59895, AAA59891, AAA59855, AAB91993, AAH31006, NP_000423, and ABC84220. The genes for these proteins has also been sequenced and characterized, see for example GenBank Accession Nos. NM_002472 and NM_000432.

The cardiomyocyte marker "actinin" is a mircrofilament protein which are the thinnest filaments of the cytoskeleton found in the cytoplasm of all eukaryotic cells. Actin polymers also play a role in actomyosin-driven contractile processes and serve as platforms for myosin's ATP hydrolysis-dependent pulling action in muscle contraction. This protein has been sequenced and characterized, see for example GenBank Accession Nos. NP_001093, NP_001095, NP_001094, NP_004915, P35609, NP_598917, NP_112267, AAI07534, and NP_001029807. The gene for this protein has also been sequenced and characterized, see for example GenBank Accession Nos. NM_001102, NM_004924, and NM_001103.

The cardiomyocyte marker "troponin" is a complex of three proteins that is integral to muscle contraction in skeletal and cardiac muscle. Troponin is attached to the protein "tropomyosin" and lies within the groove between actin filaments in muscle tissue. Tropomyosin can be used as a cardiomyocyte marker. These proteins have been sequenced and characterized, see for example GenBank Accession Nos. NP_000354, NP_003272, P19429, NP_001001430, AAB59509, AAA36771, and NP_001018007. The gene for this protein has also been sequenced and characterized, see for example GenBank Accession Nos. NM_000363, NM_152263, and NM_001018007.

"Clonal proliferation" refers to the growth of a population of cells by the continuous division of single cells into two identical daughter cells and/or population of identical cells.

As used herein, the term "comprising" is intended to mean that the compositions and methods include the recited elements, but do not exclude others. "Consisting essentially of" when used to define compositions and methods, shall mean excluding other elements of any essential significance to the combination when used for the intended purpose. Thus, a composition consisting essentially of the elements as defined herein would not exclude trace contaminants or inert carriers. "Consisting of" shall mean excluding more than trace elements of other ingredients and substantial method steps. Embodiments defined by each of these transition terms are within the scope of this invention.

A "composition" is also intended to encompass a combination of active agent and another carrier, e.g., compound or composition, inert (for example, a detectable agent or label) or active, such as an adjuvant, diluent, binder, stabilizer, buffers, salts, lipophilic solvents, preservative, adjuvant or the like. Carriers also include biocompatible scaffolds, pharmaceutical excipients and additives proteins, peptides, amino acids, lipids, and carbohydrates (e.g., sugars, including monosaccharides, di-, tri-, tetra-, and oligosaccharides; derivatized sugars such as alditols, aldonic acids, esterified sugars and the like; and polysaccharides or sugar polymers), which can be present singly or in combination, comprising alone or in combination 1-99.99% by weight or volume. Exemplary protein excipients include serum albumin such as human serum albumin (HSA), recombinant human albumin (rHA), gelatin, casein, and the like. Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like. Carbohydrate excipients are also intended within the scope of this invention, examples of which include but are not limited to monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol.

A "control" is an alternative subject or sample used in an experiment for comparison purpose. A control can be "positive" or "negative". For example, where the purpose of the experiment is to determine a correlation of an altered expression level of a gene with a particular phenotype, it is generally preferable to use a positive control (a sample from a subject, carrying such alteration and exhibiting the desired phenotype), and a negative control (a subject or a sample from a subject lacking the altered expression or phenotype). Additionally, when the purpose of the experiment is to determine if an agent effects the differentiation of a stem cell, it is preferable to use a positive control (a sample with an aspect that is known to affect differentiation) and a negative control (an agent known to not have an affect or a sample with no agent added).

The term "culturing" refers to the *in vitro* propagation of cells or organisms on or in media of various kinds. It is understood that the descendants of a cell grown in culture may not be completely identical (i.e., morphologically, genetically, or phenotypically) to the parent cell. By "expanded" is meant any proliferation or division of cells.

As used herein, the term "detectable label" intends a directly or indirectly detectable compound or composition that is conjugated directly or indirectly to the composition to be detected, e.g., N-terminal histadine tags (N-His), magnetically active isotopes, e.g., ¹¹⁵Sn, ¹¹⁷Sn and ¹¹⁹Sn, a non-radioactive isotopes such as ¹³C and ¹⁵N, polynucleotide or protein such as an antibody so as to generate a "labeled" composition. The term also includes sequences conjugated to the polynucleotide that will provide a signal upon expression of the inserted sequences, such as green fluorescent protein (GFP) and the like. The label may be detectable by itself (e.g. radioisotope labels or fluorescent labels) or, in the case of an enzymatic label, may catalyze chemical alteration of a substrate compound or composition which is detectable. The labels can be suitable for small scale detection or more suitable for high-throughput screening. As such, suitable labels include, but are not limited to magnetically active isotopes, non-radioactive isotopes, radioisotopes, fluorochromes, luminescent compounds, dyes, and proteins, including enzymes. The label may be simply detected or it may be quantified. A response that is simply detected generally comprises a response whose existence merely is confirmed, whereas a response that is quantified generally comprises a response having a quantifiable (e.g., numerically reportable) value such as an intensity, polarization, and/or other property. In luminescence or fluorescence assays, the detectable response may be generated directly using a luminophore or fluorophore associated with an assay component actually involved in binding, or indirectly using a luminophore or fluorophore associated with another (e.g., reporter or indicator) component.

Examples of luminescent labels that produce signals include, but are not limited to bioluminescence and chemiluminescence. Detectable luminescence response generally comprises a change in, or an occurrence of, a luminescence signal. Suitable methods and luminophores for luminescently labeling assay components are known in the art and described for example in Haugland, Richard P. (1996) Handbook of Fluorescent Probes and Research Chemicals (6th ed.). Examples of luminescent probes include, but are not limited to, aequorin and luciferases.

Examples of suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade Blue^{™}, and Texas Red. Other suitable optical dyes are described in the Haugland, Richard P. (1996) Handbook of Fluorescent Probes and Research Chemicals (6th ed.).

As used herein, the term "detectable label" intends a directly or indirectly detectable compound or composition that is conjugated directly or indirectly to the composition to be detected, e.g., N-terminal histadine tags (N-His), magnetically active isotopes, e.g., ¹¹⁵Sn, ¹¹⁷Sn and ¹¹⁹Sn, a non-radioactive isotopes such as ¹³C and ¹⁵N, polynucleotide or protein such as an antibody so as to generate a "labeled" composition. The term also includes sequences conjugated to the polynucleotide that will provide a signal upon expression of the inserted sequences, such as green fluorescent protein (GFP) and the like. The label may be detectable by itself (e.g. radioisotope labels or fluorescent labels) or, in the case of an enzymatic label, may catalyze chemical alteration of a substrate compound or composition which is detectable. The labels can be suitable for small scale detection or more suitable for high-throughput screening. As such, suitable labels include, but are not limited to magnetically active isotopes, non-radioactive isotopes, radioisotopes, fluorochromes, luminescent compounds, dyes, and proteins, including enzymes. The label may be simply detected or it may be quantified. A response that is simply detected generally comprises a response whose existence merely is confirmed, whereas a response that is quantified generally comprises a response having a quantifiable (e.g., numerically reportable) value such as an intensity, polarization, and/or other property. In luminescence or fluorescence assays, the detectable response may be generated directly using a luminophore or fluorophore associated with an assay component actually involved in binding, or indirectly using a luminophore or fluorophore associated with another (e.g., reporter or indicator) component.

Examples of luminescent labels that produce signals include, but are not limited to bioluminescence and chemiluminescence. Detectable luminescence response generally comprises a change in, or an occurrence of, a luminescence signal. Suitable methods and luminophores for luminescently labeling assay components are known in the art and described for example in Haugland, Richard P. (1996) Handbook of Fluorescent Probes and Research Chemicals (6th ed.). Examples of luminescent probes include, but are not limited to, aequorin and luciferases.

Examples of suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade Blue^{™}, and Texas Red. Other suitable optical dyes are described in the Haugland, Richard P. (1996) Handbook of Fluorescent Probes and Research Chemicals (6th ed.).

"Differentially expressed" intends an up- or downward expression of a gene or marker as compared to a control.

"Differentiation" describes the process whereby an unspecialized cell acquires the features of a specialized cell such as a heart, liver, or muscle cell. "Directed differentiation" refers to the manipulation of stem cell culture conditions to induce differentiation into a particular cell type. "Dedifferentiated" defines a cell that reverts to a less committed position within the lineage of a cell. As used herein, the term "differentiates or differentiated" defines a cell that takes on a more committed ("differentiated") position within the lineage of a cell. As used herein, "a cell that differentiates into a mesodermal (or ectodermal or endodermal) lineage" defines a cell that becomes committed to a specific mesodermal, ectodermal or endodermal lineage, respectively. Examples of cells that differentiate into a mesodermal lineage or give rise to specific mesodermal cells include, but are not limited to, cells that are adipogenic, leiomyogenic, chondrogenic, cardiogenic, dermatogenic, hematopoetic, hemangiogenic, myogenic, nephrogenic, urogenitogenic, osteogenic, pericardiogenic, or stromal.

As used herein, the term "differentiates or differentiated" defines a cell that takes on a more committed ("differentiated") position within the lineage of a cell. "Dedifferentiated" defines a cell that reverts to a less committed position within the lineage of a cell.

A DNA methyltransferase inhibitor is a small molecule or other agent the ability to inhibit hypermethylation, restore suppressor gene expression and exert antitumor effects in *in vitro* and *in vivo* laboratory models. Goffin and Eisenhauer (2002) Ann. Oncol. Nov. 13(11):1699-16716. One non-limiting example of such an inhibitor is N-phthalyl-L-tryptopha (C19H14N2O4, sold under the tradename RG108, Sigma-Aldrich). Additional examples include 5'-azacytidine, 5-azacytidine, antisense oligonucleotides to methylstranferase 1, e.g., MG98 (see Amato (2007) Clin. Gentourin Cancer, Dec, 5(7):422-426 and 1-(β-D-Ribofuranosyl)-2(1*H*)-pyrimidinone (a nucleoside analog of cytidine, sold under the name Zebularine (abcam^{®}).

DNA hypomethylation intends a lower than normal level of DNA methylation. Methods of determining the level of DNA methylation are known in the art, some of which are described herein.

The term effective amount refers to a concentration or amount of a reagent or composition, such as a composition as described herein, cell population or other agent, that is effective for producing an intended result, including cell growth and/or differentiation *in vitro* or in vivo, or for the treatment of a neurodegenerative condition as described herein. It will be appreciated that the number of cells to be administered will vary depending on the specifics of the disorder to be treated, including but not limited to size or total volume/surface area to be treated, as well as proximity of the site of administration to the location of the region to be treated, among other factors familiar to the medicinal biologist.

The terms effective period (or time) and effective conditions refer to a period of time or other controllable conditions (e.g., temperature, humidity for *in vitro* methods), necessary or preferred for an agent or composition to achieve its intended result, e.g., the differentiation of cells to a pre-determined cell type.

"Embryoid bodies or EBs" are three-dimensional (3D) aggregates of embryonic stem cells formed during culture that facilitate subsequent differentiation. When grown in suspension culture, EBs cells form small aggregates of cells surrounded by an outer layer of visceral endoderm. Upon growth and differentiation, EBs develop into cystic embryoid bodies with fluid-filled cavities and an inner layer of ectoderm-like cells.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in an eukaryotic cell. "Differentially expressed" as applied to a gene or marker, refers to the differential production of the mRNA transcribed from the gene or the protein product encoded by the gene. A differentially expressed gene may be overexpressed or underexpressed (a.k.a. inhibited) as compared to the expression level of a normal, non-treated or control cell. In one aspect, it refers to overexpression that is 1.5 times, or alternatively, 2 times, or alternatively, at least 2.5 times, or alternatively, at least 3.0 times, or alternatively, at least 3.5 times, or alternatively, at least 4.0 times, or alternatively, at least 5 times, or alternatively 10 times higher (i.e., and therefore overexpressed) or lower than the expression level detected in a control sample. The term "differentially expressed" also refers to nucleotide sequences in a cell or tissue which are expressed where silent in a control cell or not expressed where expressed in a control cell.

An "induced pluripotent cell" intends embryonic-like cells reprogrammed to the immature phenotype from adult cells. Various methods are known in the art, e.g., "A simple new way to induce pluripotency: Acid." Nature, 29 January 2014 and available at sciencedaily.com/releases/2014/01/140129184445, last accessed on February 5, 2014 and U.S. Patent Publication No. 2010/0041054. Human iPSCs also express stem cell markers and are capable of generating cells characteristic of all three germ layers.

"Isoxazole" is a class of compounds found in some natural products, such as ibotenic acid, as well as a number of drugs, including a COX-2 inhibitor, and furoxan, a nitric oxide donor. Isoxazoles are useful isosteres of pyridine, and have been found to inhibit voltage-gated sodium channels to control pain, enable the construction of tetracycline antibiotic derivatives, and as treatments for depression. Compounds of this class available from Sigma-Aldrich and methods to synthesize such are known in the art as described for example in U.S. Patent Nos. 5,059,614 and 8,318,951 and PCT Publication No. WO 1999/002507.

As used herein, the term "isoxazole-like compound" or "similar compound" intends an agent or small molecule that has the same functional property of the isoxazole as disclosed herein. Non-limiting examples include Cardionogen; CDNG1/vuc230, CDNG2/vuc198, and CDNG3/vuc247 (see Terri et al. (2011) Chem Biol., Dec. 23 18(12):1658-1668).

The term "isolated" as used herein refers to molecules or biological or cellular materials being substantially free from other materials, e.g., greater than 70%, or 80%, or 85%, or 90%, or 95%, or 98%. In one aspect, the term "isolated" refers to nucleic acid, such as DNA or RNA, or protein or polypeptide, or cell or cellular organelle, or tissue or organ, separated from other DNAs or RNAs, or proteins or polypeptides, or cells or cellular organelles, or tissues or organs, respectively, that are present in the natural source and which allow the manipulation of the material to achieve results not achievable where present in its native or natural state, e.g., recombinant replication or manipulation by mutation. The term "isolated" also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides, e.g., with a purity greater than 70%, or 80%, or 85%, or 90%, or 95%, or 98%. The term "isolated" is also used herein to refer to cells or tissues that are isolated from other cells or tissues and is meant to encompass both cultured and engineered cells or tissues.

As used herein, the "lineage" of a cell defines the heredity of the cell, i.e. its predecessors and progeny. The lineage of a cell places the cell within a hereditary scheme of development and differentiation.

A "multi-lineage stem cell" or "multipotent stem cell" refers to a stem cell that reproduces itself and at least two further differentiated progeny cells from distinct developmental lineages. The lineages can be from the same germ layer (i.e. mesoderm, ectoderm or endoderm), or from different germ layers. An example of two progeny cells with distinct developmental lineages from differentiation of a multilineage stem cell is a myogenic cell and an adipogenic cell (both are of mesodermal origin, yet give rise to different tissues). Another example is a neurogenic cell (of ectodermal origin) and adipogenic cell (of mesodermal origin).

A "parthenogenetic stem cell" refers to a stem cell arising from parthenogenetic activation of an egg. Methods of creating a parthenogenetic stem cell are known in the art. See, for example, Cibelli et al. (2002) Science 295(5556):819 and Vrana et al. (2003) Proc. Natl. Acad. Sci. USA 100(Suppl. 1)11911-6 (2003).

The term "phenotype" refers to a description of an individual's trait or characteristic that is measurable and that is expressed only in a subset of individuals within a population. In one aspect of the invention, an individual's phenotype includes the phenotype of a single cell, a substantially homogeneous population of cells, a population of differentiated cells, or a tissue comprised of a population of cells.

As used herein, a "pluripotent cell" defines a less differentiated cell that can give rise to at least two distinct (genotypically and/or phenotypically) further differentiated progeny cells. In another aspect, a "pluripotent cell" includes an Induced Pluripotent Stem Cell (iPSC) which is an artificially derived stem cell from a non-pluripotent cell, typically an adult somatic cell, that has historically been produced by inducing expression of one or more stem cell specific genes. Such stem cell specific genes include, but are not limited to, the family of octamer transcription factors, i.e. Oct-3/4; the family of Sox genes, i.e. Sox1, Sox2, Sox3, Sox 15 and Sox 18; the family of Klf genes, i.e. Klf1, Klf2, Klf4 and Klf5; the family of Myc genes, i.e. c-myc and L-myc; the family of Nanog genes, i.e. OCT4, NANOG and REX1; or LIN28. Examples of iPSCs are described in Takahashi et al. (2007) Cell advance online publication 20 November 2007; Takahashi & Yamanaka (2006) Cell 126:663-76; Okita et al. (2007) Nature 448:260-262; Yu et al. (2007) Science advance online publication 20 November 2007; and Nakagawa et al. (2007) Nat. Biotechnol. Advance online publication 30 November 2007.

A "precursor" or "progenitor cell" intends to mean cells that have a capacity to differentiate into a specific type of cell. A progenitor cell may be a stem cell. A progenitor cell may also be more specific than a stem cell. A progenitor cell may be unipotent or multipotent. Compared to adult stem cells, a progenitor cell may be in a later stage of cell differentiation. An example of progenitor cell includes, without limitation, a progenitor nerve cell.

The term pharmaceutically acceptable carrier (or medium), which may be used interchangeably with the term biologically compatible carrier or medium, refers to reagents, cells, compounds, materials, compositions, and/or dosage forms that are not only compatible with the cells and other agents to be administered therapeutically, but also are, within the scope of sound medical judgment, suitable for use in contact with the tissues of human beings and animals without excessive toxicity, irritation, allergic response, or other complication commensurate with a reasonable benefit/risk ratio. Pharmaceutically acceptable carriers suitable for use in the present invention include liquids, semi-solid (e.g., gels) and solid materials (e.g., cell scaffolds and matrices, tubes sheets and other such materials as known in the art and described in greater detail herein). These semi-solid and solid materials may be designed to resist degradation within the body (non-biodegradable) or they may be designed to degrade within the body (biodegradable, bioerodable). A biodegradable material may further be bioresorbable or bioabsorbable, i.e., it may be dissolved and absorbed into bodily fluids (water-soluble implants are one example), or degraded and ultimately eliminated from the body, either by conversion into other materials or breakdown and elimination through natural pathways.

As used herein, a "pluripotent cell" defines a less differentiated cell that can give rise to at least two distinct (genotypically and/or phenotypically) further differentiated progeny cells.

A population of cells intends a collection of more than one cell that is identical (clonal) or non-identical in phenotype and/or genotype.

The term "propagate" means to grow or alter the phenotype of a cell or population of cells. The term "growing" refers to the proliferation of cells in the presence of supporting media, nutrients, growth factors, support cells, or any chemical or biological compound necessary for obtaining the desired number of cells or cell type. In one embodiment, the growing of cells results in the regeneration of tissue. In yet another embodiment, the tissue is comprised of cardiac progenitor cells or cardiac cells.

A "skeletal myoblast (SM)" is an immature cell that can be isolated from between the basal lamina and sarcolemma. They account for 2-5% of sub-laminar nuclei of mature skeletal muscle. Skeletal myoblasts are activated in response to muscle damage or disease-induced muscle degeneration. Skeletal myoblasts express desmin, CD56, Pax3, Pax7, c-met, myocyte nuclear factor, M-cadherin, VCAM1, N-CAM, CD34, Leu-19, and syndecan 3 and 4. Activated skeletal myoblasts first express Myf-5 and/or MyoD, and finally myogenin and MRF4 as the cells differentiate into multinucleated myotubes.

As used herein, the term "small juvenile stem cells (SJSCs)" intends stem cells isolated from aged bone marrow-derived stem cells (BMSCs) with high proliferation and differentiation potential. See Iqura et al. (2013) 305(8):H1354-62. SJSCs express mesenchymal stem cell markers, CD29(+)/CD44(+)/CD59(+)/CD90(+), but are negative for CD45(-)/CD117(-) as examined by flow cytometry analysis. SJSCs show higher proliferation, colony formation, and differentiation abilities compared with BMSCs. They also are reported to significantly express cardiac lineage markers (Gata-4 and myocyte-specific enhancer factor 2C) and pluripotency markers (octamer-binding transcription factor 4, sex-determining region Y box 2, stage-specific embryonic antigen 1, and Nanog) as well as antiaging factors such as telomerase reverse transcriptase and sirtuin 1.

As used herein, "stem cell" defines a cell with the ability to divide for indefinite periods in culture and give rise to specialized cells. At this time and for convenience, stem cells are categorized as somatic (adult), embryonic or induced pluripotent stem cells. A somatic stem cell is an undifferentiated cell found in a differentiated tissue that can renew itself (clonal) and (with certain limitations) differentiate to yield all the specialized cell types of the tissue from which it originated. An embryonic stem cell is a primitive (undifferentiated) cell from the embryo that has the potential to become a wide variety of specialized cell types. Non-limiting examples of embryonic stem cells are the HES2 (also known as ES02) cell line available from ESI, Singapore and the H1 or H9 (also known as WA01) cell line available from WiCell, Madison, WI. Pluripotent embryonic stem cells can be distinguished from other types of cells by the use of markers including, but not limited to, Oct-4, alkaline phosphatase, CD30, TDGF-1, GCTM-2, Genesis, Germ cell nuclear factor, SSEA1, SSEA3, and SSEA4. An iPSC is an artificially derived stem cell from a non-pluripotent cell, typically an adult somatic cell, produced by inducing expression of one or more stem cell specific genes, or as claimed herein, manipulation of culture conditions to establish an embryonic-like phenotype. Insofar as the mentioned hESC were obtained by the destruction of a human embryo or from a cell line that had been established by the destruction of a human embryo, such hESC do not form part of the claimed invention and are mentioned for reference purposes only.

A "subject," "individual" or "patient" is used interchangeably herein, and refers to a vertebrate, preferably a mammal, more preferably a human. Mammals include, but are not limited to, murines, rats, simians, bovines, canines, felines, humans, farm animals, sport animals and pets.

"Substantially homogeneous" describes a population of cells in which more than about 50%, or alternatively more than about 60 %, or alternatively more than 70 %, or alternatively more than 75 %, or alternatively more than 80%, or alternatively more than 85 %, or alternatively more than 90%, or alternatively, more than 95 %, of the cells are of the same or similar phenotype. Phenotype can be determined by a pre-selected cell surface marker or other marker.

As used herein, the terms "treating," "treatment" and the like are used herein to mean obtaining a desired pharmacologic and/or physiologic effect. The effect can be prophylactic in terms of completely or partially preventing a disorder or sign or symptom thereof, and/or can be therapeutic in terms of a partial or complete cure for a disorder and/or adverse effect attributable to the disorder. Examples of "treatment" include but are not limited to: preventing a disorder from occurring in a subject that may be predisposed to a disorder, but has not yet been diagnosed as having it; inhibiting a disorder, i.e., arresting its development; and/or relieving or ameliorating the symptoms of disorder, e.g., cardiac arrhythmia. As is understood by those skilled in the art, "treatment" can include systemic amelioration of the symptoms associated with the pathology and/or a delay in onset of symptoms such as chest pain. Clinical and sub-clinical evidence of "treatment" will vary with the pathology, the individual and the treatment.

### Descriptive Embodiments

### Chemically Modified Stem Cells

This disclosure describes a chemically modified induced pluripotent stem cell (iPSC) characterized by DNA hypomethylation. The cell can additionally be characterized by overexpression of one or more cardiac gene or marker selected from the group of Gαi, mir-133, mir-762, CCL7, CXCR2, CXC5, integral membrane protein 2A, and ephrin A3. Nkx 2.5, GATA4, αMHC, Sarcomeric actin, Gαi, mir-133, mir-762, CCL7, CXCR2, CXC5, integral membrane protein 2A, and ephrin A3. The cell may under express one or more pluripotent genes or markers, non-limiting examples of such include one or more of miR-290-295 cluster, let-7 family, Max and may under express one or more DNA methyltransferase genes Dnmt1, Dnmt3b. The one or more cardiac gene or marker can be Gαi. The one or more cardiac gene or marker can be overexpressed at least 1.5 fold, or alternatively at least 2 fold, or alternatively at least 3 fold, over that of a control cell. The cell can be characterized by underexpression of one or more other cardiac gene or marker selected from the group of miR-290 clustet, miR-574-5P, let-7 family, Dnmt1, Dnmt3b, and Max. The one or more cardiac gene or marker can be underexpressed by at least 1.5 fold, or alternatively at least 2 fold, or alternatively at least 3 fold, under that of a control cell. Methods to identify and quantitate genes and markers are known in the art and described herein to supplement well known methods.

Gαi is a heterotrimeric G protein subunit that inhibits the product of cAMP from ATP. An exemplary sequence is provided under GenBank Ref.: NM_002069 and UnProt P63096. Antibodies that recognize this marker are commercially available from Santa Cruz Biotechnology.

Mir-133 refers to a microRNA that has been linked to an immature or undifferentiated phenotype. Methods to detect such include, for example, microarray- RT-PCR and RNA-seq. Commercially available kits to mir-133 is available from EMD Millipore (SmartFlare^{™} Detection Probes) which allow for the detection of miRNA in live cells.

Mir-762 is a non-coding RNA that has been linked to post-transcriptional regulation of gene expression in multicellular organisms. The miR-762 human sequence is reported under Accession No. MI0003892 (last accessed on April 16, 2014). The murine sequence is reported under NR_030428.1 (see ncbi.nlm.nih.gov/gene/79103, last accessed on April 16, 2014). Methods to detect such are known in the art and kits are commercially available from, for example, Origene (Mir762, see origene.com, last accessed on April 16, 2014).

As used herein, the term "pluripotent gene or marker" intends an expressed gene or protein that has been correlated with an immature or undifferentiated phenotype, e.g., Oct ¾, Sox2, Nanog, c-Myc and LIN-28. Methods to identify such are known in the art and systems to identify such are commercially available from, for example, EMD Millipore (MILLIPLEX^{®} Map Kit).

The miR-290-295 cluster is a pluripotent cluster codes for a family of microRNAs (miRNAs) that are expressed de novo during early embryogenesis and are specific for mouse embryonic stem cells (ESC) and embryonic carcinoma cells (ECC). Such are known in the art and described, for example, in Lichner et al. (2011) Differentiation, Jan. 81(1):11-24.

Chemokine (C-C motif) ligand 7 (CCL7) is a small cytockine previously known as monocyte-specific chemokine 3 (MCP3). The protein sequence is available under Accession number NP_006264 and the murine sequence is available under NP_038682 (see also ncbi.nlm.nih.gov/gene/6354, last accessed on April 16, 2014). An antibody and kit to detect CCL7 is available from Sino Biological Inc.

CXCR2 chemokine receptor 2 (CXCR2) is a protein encoded by by this gene is a member of the G-protein-coupled receptor family. This protein is a receptor for interleukin 8 (IL8). It binds to IL8 with high affinity, and transduces the signal through a G-protein activated second messenger system. This receptor also binds to chemokine (C-X-C motif) ligand 1 (CXCL1/MGSA). Information regarding the protein and its gene is found on nchbi.nlm.nih.gov/gene/3579 (last accessed on April 16, 2014).

Integral membrane protein 2A is a stem cell marker. The sequence of the human gene is reported at UniProtKB (O43736) and the murine sequence is reported at Q61500 (uniprot.org/uiprot, last accessed on April 16, 2014).

DNA (cytosine-5)-methyltransferase 1 is an enzyme that is encoded by the DNMT1 gene. The complete sequence of the protein and its gene is available at genecards.org/cgi-bin/carddisp.pl?gene=DNMT1, last accessed on April 16, 2014. Antibodies to detect the protein are commercially available, e.g., from Cell Signaling Technologies (DNMT1 (D63A6) XP^{®} Rabbit mAb #5032). DNA (cytosine-5)-methyltransferase 3 is an enzyme that is encoded by the DNMT3 gene.

EFNA3 or ephrin A3 is a protein receptor. The human protein sequence is reported at ncbi.nlm.nih.gov/gene/1944. Antibodies useful for the detection and analysis of the protein are available from R&D Systems and Santa Cruz Biotechnology.

"Let-7" refers to a family of microRNAs. The sequences are reported at the miRBase at mirbase.org/cgi-bin/mirna_summary.pl?fam=MIPF000002, last accessed on April 16, 2014. Methods for detecting such are known in the art, e.g., U.S. Patent Application No. 2014/0005251.

Max is a pluripotency marker that binds MYC. See Chappell et al. (2013) Genes & Dev. 27:725-733.

The cell can be from any animal species, such as a mammal, e.g., an equine, a murine, a bovine, a canine, a feline, or a human patient.

The iPS cell is derived from any suitable parent cell. Non-limiting examples of such include, without limitation, a cell selected from the group consisting of a bone marrow cell, a myoblast, a skin fibroblast, a cord blood cell, an adult peripheral blood, a small juvenile stem cell (SJST), or a mononuclear cell.

Methods to derive or prepare an iPSC from these parent cell types are known in the art. In one aspect, the iPSC was created by a method comprising contacting the parent cell with an effective amount of a DNA methyltransferase inhibitor to upregulate Oct4. Non-limiting examples of DNA methyltransferease inhibitors include 5'-azacytidine, 5-aza-2'-deoxycytidine, MG98, zebularine and RG108. In another aspect, the iPS cell was created by a method that excludes the insertion of exogenous genes into the parent cell.

The parent cells can be cardiac progenitor cell prepared by preconditioning the cells with electrical stimulation. The parent cell can be a stem cell that expresses Sea 1 and pluripotency and cardiac genes as described herein. The cells are subsequently contacted with isoxazole or an isoxazole similar compound as described herein.

The parent cell can be an small juvenile stem cell that is not modified or programmed to an iPSC. The SJST can be treated with the isoxazole or isoxazole similar compound and used diagnostically, in research or therapeutically. Also described is a method to direct an immature or progenitor cell, e.g., a SJST cell, a circulating blood or heart derived stem cell) to a cardiac progenitor phenotype by contacting the cell with an effective amount of an isoxazole or isoxazole similar compound as described herein.

The chemically modified iPS cell as described herein is prepared by contacting the cell with an effective amount of an isoxazole or isoxazole similar compound, e.g., an amount that is selected from the group of from about 0.5 to about 30 uM; from about 0.5 to about 25 uM; from about 12 to about 25 uM and from about 0.5 uM to about 20 uM.

While methods to prepare an iPS cell are known in the art, Applicant has determined that an iPS cell created by a method comprising contacting a parent cell with an effective amount of a DNA methyltransferase inhibitor, e.g., 5'-azacytidine or RG108 (Sigma-Aldrich) to upregulate Oct4 is particularly useful. The iPS cells can additionally prepared by a method that excludes the insertion of exogenous genes into the parent cell thereby enhancing the safety of the cells for clinical use.

Also described is a population of cultured cells as described herein, by culturing the chemically modified cells to expand the cells as described herein. The population can be substantially homogenous, e.g., at least 70 % identical in phenotype, or alternatively at least 75 % identical in phenotype, or alternatively at least 80 % identical in phenotype, or alternatively at least 85% identical in phenotype, or alternatively at least 90% identical in phenotype, or alternatively at least 95% identical in phenotype, or alternatively at least 98% identical in phenotype or alternatively, a clonal population of cells. The population can be a clonal population. The cells can be cultured under conditions that favor differentiation into a particular cell type, e.g., a cardiac cell. These culturing conditions are known in the art.

The cells and populations of cells can be further modified for therapeutic or research use, for by example, further comprising a detectable label or exogenous polynucleotide or polypeptide. Methods and appropriate polynucleotides for therapeutic use are described in Durrani et al. (2010) Skeletal myoblasts for cardiac repair, Regen. Med. 5(6):919-932.

### Method for Preparing the Cells

Described is a method for preparing a cardiac lineage cell from a stem cell, comprising contacting the stem cell with an effective amount of an isoxazole or isoxazole similar compound. Stem cells useful in the method include, without limitation, a one or more of an iPS cell, such as an iPS cell derived from a bone marrow cell, a myoblast, a skin fibroblast, a cord blood cell, an adult peripheral blood, a SJSC, or mononuclear cell. Methods to derive or prepare an iPSC from these parent cell types are known in the art. The cells prepared by this method are characterized in overexpressing one or more cardiac gene or marker, e.g., Nkx 2.5, GATA4, αMHC, Sarcomeric actin, Gαi, mir-133, mir-762, CCL7, CXCR2, CXC5, integral membrane protein 2A, or ephrin A3 and/or underexpressing one or more cardiac gene or marker, e.g., miR-290-295 cluster, let-7 family, Dnmt1, Dnmt3b, and Max, each as compared to a control cell such as a cell that has not been contacted with or exposed to the isoxazole or isoxazole similar compound. In one aspect, the over- or underexpression is at least 1.5 fold over- or under- that of the control cell.

The cell may be a small juvenile stem cell that is not modified or programmed to an iPSC. In this aspect, the SJST is treated with the isoxazole or isoxazole similar compound and used diagnostically, in research or therapeutically. Also described is a method to direct an immature or progenitor cell, e.g., a SJST cell, (contained with a population of circulating blood or heart derived stem cells) to a cardiac progenitor phenotype by contacting the cell with an effective amount of an isoxazole or isoxazole similar compound as described herein.

The method may comprise, or alternatively consist essentially of, or yet further consist of, contacting the cells with the effective amount of the isoxazole or isoxazole similar compound for at least 3 days, or alternatively at least 4 days, or alternatively at least 5 days, or alternatively at least 6 days, or alternatively at least 7 days, in DMEM F12 supplemented with from about 10 % to about 30%, or alternatively 20% Knockout Serum Replacement (KSR; Invitrogen, USA), and from about 0.05 mM to about 0.2 mM, or about 0.1 mM MEM Non-Essential Amino Acids solution (Invitrogen, CA, USA), and from about 0.1 mM to about 0.3 mM or about 0.2 mM L- glutamine (Invitrogen, USA); and from about 0.05 mM to about 0.2 mM or alternatively from about 0.1 mM β- mercaptoethanol (Invitrogen, CA, USA); and from about 750 U/ml to about 1250 U/ml or alternatively from about 1000 U/ml LIF (Millipore); and an effective amount of 0.5% penicillin and streptomycin. The cells are then kept in the media without drug for five days. The media is changed every day otherwise due to increase in cell number the pH of the media get changed and effect the cell survival and gene expression. An effective amount of the isoxazole or isoxazole similar compound comprises, or alternatively consists essentially of, or yet further consists of from about 0.3 to about 30 uM; or from about 0.5 to about 25 uM; or from about 12 to about 25 uM; or from about 0.5 uM to about 20 uM. As used herein, isoxazole or isoxazole similar intends a class of compounds as described above.

Methods to identify and quantitate genes and markers are known in the art and described herein to supplement well known methods.

The cell can be from any animal species, such as a mammal, e.g., an equine, a murine, a bovine, a canine, a feline, or a human patient.

The contacting can be performed *in vitro,* e.g., in a tissue culture dish or plate as described herein or *in vivo,* by administering an effective amount of the isoxazole or isoxazole similar compound to a cell culture or *in vivo* by administering an effective amount of the compound or alternatively, compound and iPSCs to a patient or subject in need of such treatment. Methods for administering, systemically or locally, such are described below. The compounds and/or cells can be combined with a pharmaceutically acceptable carrier for ease of use.

The stem cell contacted with the compound is an iPS cell. Methods to generate iPS cell from terminally differentiated cells are known in the art, e.g., by a method comprising contacting the parent cell with an effective amount of a DNA methyltransferase inhibitor, e.g., RG108 (Sigma-Aldrich) to upregulate Oct4. In another aspect, the iPS cell is created by a method that excludes the insertion of exogenous genes into the parent cell.

Also described is an isolated cell prepared by a method as described herein by further comprising isolating the cells. Yet further, the method further may comprise culturing the cells to prepare a population of cells. The population may be cultured under conditions to prepare a substantially homogenous, e.g., at least 70 % identical in phenotype, or alternatively at least 75 % identical in phenotype, or alternatively at least 80 % identical in phenotype, or alternatively at least 85% identical in phenotype, or alternatively at least 90% identical in phenotype, or alternatively at least 95% identical in phenotype, or alternatively at least 98% identical in phenotype. The method may further comprise culturing the cells under conditions that favor clonal expansion of the cells to a clonal population. The cells may be cultured under conditions that favor differentiation into a particular cell type, e.g., a cardiac cell. These culturing conditions are known in the art.

The methods can be further modified by inserting into the cells and populations of cells a detectable label or exogenous polynucleotide or polypeptide. Methods and appropriate polynucleotides for therapeutic use are described in Durrani et al. (2010) Skeletal myoblasts for cardiac repair, Regen. Med. 5(6):919-932.

### Compositions

Also described are compositions containing the cells, population of cells and/or differentiated cells in combination with a carrier, such as a biocompatible scaffold or a pharmaceutically acceptable carrier. The composition may be intended for therapeutic use and therefore, an effective amount of the modified cell, population of cells or differentiated cells can be provided, alone or in combination with the isoxazole or isoxazole similar compound, in the composition.

### Uses of the Cells and Cell Populations

Also described is a method for restoring cardiac function in a tissue or host in need thereof. This and other therapeutic uses are described herein.

Described are methods for one or more of: regenerating cardiac muscle tissue that in one aspect, is scar tissue in the damaged or diseased heart; improving cardiac function or for treating a cardiac disease or condition in a patient in need thereof. The methods comprise contacting the tissue to be regenerated with an effective amount of isoxazole or isoxazole similar compound or by administering to a subject in need thereof, and/or an effective amount of the chemically modified cell or population of chemically modified cells described above. The isolated cell, e.g., an iPS cell or other progenitor cell may be locally administered within an effective amount of isoxazole or isoxazole similar compound. The treated iPS cells may have been differentiated into myocytes forming myofibers in the scar tissue of the heart. The cells can be autologous or allogeneic to the host or patient. The subject and cells can be any species as described herein.

Also described is a method for regenerating cardiac muscle tissue in a suitable host by administering to the host an effective amount of the chemically modified cell or population of chemically modified cells as described above. The cells can be autologous or allogeneic to the host or patient. The subject and cells can be any species as described above.

Also described is a method comprising administering to a patient in need thereof an effective amount of an iPS cell and an effective amount an isoxazole or isoxazole similar compound. Administration is local to the site of damage, and can include direct injection of the cells and isoxazole or isoxazole similar compound into the heart of the patient.

The stem cells or iPS cells can be administered in the form of a cardiac patch derived from stem cells (e.g., adult or bone marrow derived progenitors cells, iPS cells, small juvenile stem cells and /or engineered tissue cardiac patch transplantation for the heart repair in heart diseases). Thus, the stem cells may comprise very small juvenile stem cells that are present in the bone marrow (that are comprised within bone marrow stem cells) and/or peripheral blood cells (that are comprised within circulating blood and heart- derived stem cells). These cells are with significantly high proliferative and differentiation potentials and can also be easily reprogrammed and/or converted to cardiac progenitors with isoxazole or isoxazole similar compounds. Thus, this method would not require the derivation of iPS cells for safe cell transplantation.

Patients suitably treated by this method include those suffering from a disease or disorder associated with cardiac malfunction including, but not limited to, congestive heart failure, isolated diastolic heart failure, myocardial infarction, and cardiac arrhythmia. There are several forms of cardiac arrhythmia that can be treated including, but not limited to, sick sinus syndrome, bradyarrhythmia, abnormal sinus node function, atrioventricular block, and atrial and ventricular tachyarrhythmia.

Local or systemic administration, by use of a catheter or cardiac patch, of the cells or compositions can be effected in one dose, continuously or intermittently throughout the course of treatment. Methods of determining the most effective means and dosage of administration are known to those of skill in the art and will vary with the composition and/or cells used for therapy, the purpose of the therapy and the subject being treated. Single or multiple administrations can be carried out with the dose level and pattern being selected by the treating physician. Suitable dosage formulations and methods of administering the cell or agents are known in the art. The cells and compositions as described herein can be administered in combination with other treatments.

The cells and populations of cell can be administered to the host using methods known in the art and described, for example, in U.S. Patent No. 6,638,369 and can be allogeneic or autologous to the patient. This administration of the cells or compositions as described herein can be done to treat disease as noted herein, and to produce an animal model of the desired disease, disorder, or condition for experimental and screening assays.

### Screening Assays

Also described herein are methods for screening various agents that modulate cardiac function. For the purposes of this invention, an "agent" is intended to include, but not be limited to a biological or chemical compound such as a simple or complex organic or inorganic molecule, a peptide, a protein (*e.g*. antibody), a polynucleotide (e.g. anti-sense) or a ribozyme. A vast array of compounds can be synthesized, for example polymers, such as polypeptides and polynucleotides, and synthetic organic compounds based on various core structures, and these are also included in the term "agent." In addition, various natural sources can provide compounds for screening, such as plant or animal extracts, and the like. It should be understood, although not always explicitly stated that the agent is used alone or in combination with another agent, having the same or different biological activity as the agents identified by the inventive screen.

To practice the screening method *in vitro,* suitable cell cultures or tissue cultures containing the modified cell(s) are first provided. When the agent is a composition other than a DNA or RNA, such as a small molecule as described above, the agent can be directly added to the cell culture or added to culture medium for addition. As is apparent to those skilled in the art, an "effective" a mount must be added which can be empirically determined. When agent is a polynucleotide, it can be directly added by use of a gene gun or electroporation. Alternatively, it can be inserted into the cell using a gene delivery vehicle or other method as described above. Positive and negative controls can be assayed to confirm the purported activity of the drug or other agent.

### Exemplary Embodiments

Applicant has discovered that iPSc can be chemically induced to DNA hypomethylation causing upregulation of cardiac genes and allowing successful propagation in the diseased heart with no or limited chances for tumorgenecity.

Skeletal myoblasts (SMs) purified from young male Oct3/4-GFP⁺ transgenic mouse were treated with DNA methyltransferase inhibitor 500µM RG-108 in 0.5% DMSO in knock out DMEM for 5-days. Two weeks later, GFP⁺ colonies of SM derived iPS cells (SiPS) expressing GFP and morphological features of mouse embryonic stem cells were isolated and propagated *in vitro.* SiPS were positive for alkaline phosphatase activity, expressed SSEA1 and displayed a panel of pluripotency markers (Figure 1) similar to ES cells and developed teratomas in nude mice. Although the research described herein was conducted in a murine model, the use of the same markers and agents will provide similar if not identical results in human cells and tissue.

In order to direct SiPS towards cardiac lineage cells, they were treated with a small molecule (Isoxazole, 20 uM, Sigma-Aldrich) for five days and analyzed for DNA methyltransferase (Dnmt) activity, cell proliferation, and cardiac gene expression. DNMT activity was completely abolished with 95% reduction in global DNA methylation in small molecule treated SiPS (Figure 2). These SiPS showed increased proliferative activity (p<0.01 vs. non treated Sips) evaluated by cell proliferation assay and also become tolerant to apoptosis (Figure 3) an important consideration for preventing cell death in the ischemic environment. Small molecule treated IPS cells show a significant decrease in cytochrome c translocation to cytoplasm as compared to the untreated IPS cells (Figure 4).

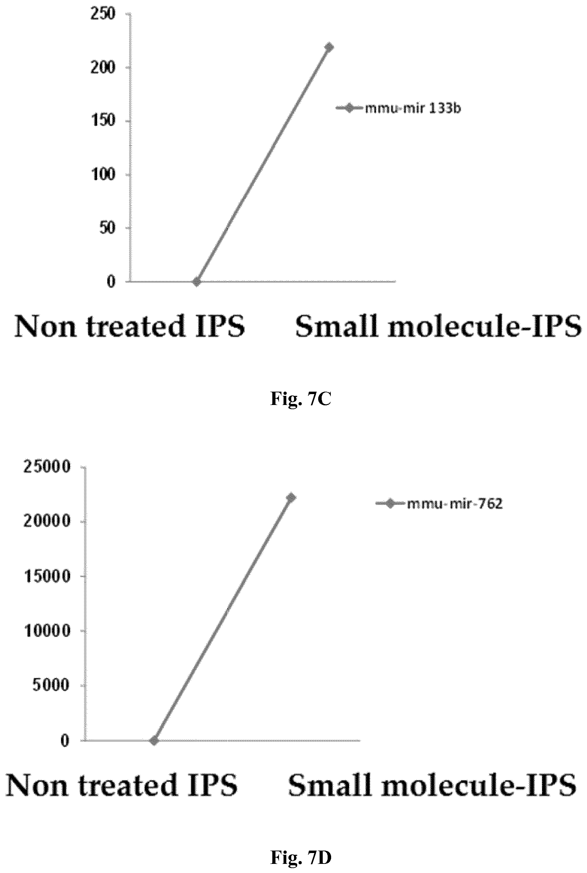

RT PCR analysis showed cardiomyocyte-like gene expression profile with significant upregulation of Nkx2.5 (p<0.01 vs non treated IPS; see Figure 5). Approximately 60% treated iPS cells were positive for NKX2.5. Given that posttranscriptional regulation is crucial for gene expression and cell survival molecular phenotypic analysis was performed, Affymetrix array-based gene expression profiling further confirmed 2-3 folds downregulation of Dnmt1, Dnmt3b and Max gene associated protein which were associated with global DNA hypomethylation and myc dependent cell transformation (see Table 1). Additionally, there was a 2-3 fold concomitant upregulation in the CCL7, CXCR2, CXCR5, integral membrane protein 2A, and ephrin A3. (Figure 6). These were associated with DNA synthesis, cell proliferation, cell matrix interaction and chemoattraction. miR microarray analysis showed upregulation of cardiac specific mir -133, 762 and down regulation of pluripotency associated miR-290 cluster, miR-574-5p and let-7 family (see Figure 7). Western blot analysis showed significant upregulation of Gαi protein levels as compared to untreated IPS cells (see Figure 8).

The SiPs treated with the small molecule were stained with PKH 26 for locating them in the heart and transplanted in the myocardium after 30 minutes of coronary artery ligation for 6 weeks. Before harvesting the hearts for visualizing the fate of transplanted SiPs, cardiac function was monitored with echocardiography. The treated SiPs were differentiated into myocytes forming myofibers in the scar tissue. These are significant findings have not been reported previously.

Applicant also discovered that isoxazoles induced DNA hypomethylation and myc dependent cell transformation in the iPS cells and were associated with DNA synthesis, cell proliferation, cell matrix interaction and chemotexis. The isoxazoles compound upregulated the CXC chemokine receptors and integral membrane proteins, Epherin family and related receptors that specifically involved in the development of erythropoiesis and angiomyogenesis.

Applicant has reported that small molecule treatment induced overexpression of several cardiac genes, microRNAs, and bioactive molecules. Their treatment of infarcted hearts led to extensive regeneration of infracted hearts and their enhanced survival as well. Based on Applicant's previous work on bone marrow derived stem cells (Circ Res 98: 1414-1421; 2006;) it is known that the cells release a number of paracrine factors (cytokines and growth factors) and, without being bound by theory, it is Applicant's belief that small molecule-treated induced pluripotent stem cells also release bioactive factors important in cell survival and angiomyogenesis.

Applicant further discovered that isoxazoles upregulated cardiac specific mir -133, mir-762 and down regulation of pluripotency associated miR-290 cluster and let-7 family in iPS cells as compared to untreated iPS cells.

Thus, the above reports that small molecule-mediated modification of iPS cells can grow in the infarcted heart and replace the scar tissue with working myocytes coupled together with electrical connections (gap junctions). The sulfonyl-hydrazone family of small molecules can induce cardiac genes in iPS cells derived from myoblasts. These small molecules are known to induce muscle differentiation in Notch activated epicardium derived progenitors (7). Small molecule induced iPS cells were engrafted in post ischemic model and improved global cardiac function compared to non-treated iPS cells. Recovery of cardiac function was dependent on the survival of the iPS cell-derived progenitors cells. Small molecules are innovative in inducing the tissue-specific gene expression in iPS cell towards tissue differentiation, as well as determining the temporal and spatial patterns of development.

Applicant has discovered that the induced pluripotent stem cells, if treated with the appropriate small molecule, can pharmacologically inhibit the DNA methylation, hence a critical player in the regulation of cardiac developmental genes. Without being bound by theory, the effect of small molecule on epherin family may manipulate the process of hematopoietic progenitors generation and could be beneficial for clinical hematopoietic malignancies. Also epherin receptors and ligands regulate differentiation, proliferation, and migration of various cell types during development and enhance angiogenesis.

Applicant further observed that treatment of induced pluripotent stem cells with the small molecule caused significant upregulation of integral membrane protein 2A which is associated with myogenesis.

Applicant also discovered that isoxazoles upregulated cardiac specific mir -133, mir-762 and down regulation of pluripotency associated miR-290 cluster and let-7 family in iPS cells as compared to nontreated iPS cells.

Eric Oslon in U.S. Patent No. 8,318,951 (`951 Patent) previously reported the use of these compounds in neurogenesis, epicardial progenitors cells. Regardless of these studies, the findings reported herein are unique and innovative in that the findings address the small molecule induced epigenetic changes that can be manipulated for rendering the iPS cells for propagation in the infarcted heart. This work emphasizes the iPS cells which can be made from a patient cell (in this case skeletal muscle) and reintroduced into the same patient after pretreatment with small molecule. These findings are distinct from the work of Oslon since iPS cells of the present disclosure were therapeutically predesigned (or pretreated) for propagation in the scar tissue of the mice heart after coronary artery ligation or heart attack. There was significant regeneration in the scar tissue by iPS cells. On the other hand, Olson injected the drug into live mice to act on heart progenitors present in the heart. It is Applicant's belief that the drug worked on different cells of the heart. Here, the composition and methods predesigned the iPS cells into cardiac progenitors in the dish and then reintroduced into damaged hearts.

Applicant also found that IPS pretreatment with small molecule reduced the apoptosis which is also novel finding. For example, less apoptosis was observed in small molecule induced iPS cells as compared to non-induced iPS cells. In this study apoptosis was evaluated by using Tunnel assay and cytochrome c translocation. The results reported in the `951 Patent were generated only using the Trypan blue exclusion assay for cell viability not cell apoptosis which is critical in cell survival under ischemic condition.

Applicant also found that DNA methyltransferase (DNMT) activity was completely abolished in the chemically modified IPS cells. There was 2-3 folds downregulation of Dnmt1, Dnmt3b and Max gene associated protein in small molecule induced iPS cells which are associated with DNA hypomethylation and cell transformation. There was 2-3 folds upregulation of CCL7, CXCR2, CXCR5, integral membrane protein 2A, and EphrinA3 which are associated with cell mobilization, chemotaxis, cancer and erythropoiesis was also observed. Applicant further observed upregulation of cardiogenic specific mir -133, mir-762 and down regulation of pluripotency associated miR-290-295 cluster and let-7 family which confirms the induction of the cardiac regulatory genes by Micro RNAs by down reulating the pluripotency genes.

In addition, the `951 Patent didn't observe Gα protein level, as compared to the work reported here, which did observe the Gα protein level upregulation which was blocked when the chemically modified iPS cells were treated with GPCR blocker and abolished the all *in vitro* effect in small molecule induced iPS cells. Oslon previously has reported the use of these compounds in neurogenesis, epicardial progenitors cells. Regardless of the previous studies, it is Applicant's belief that the current findings are unique and innovative that address the small molecule induced epigenetic changes that can be manipulated for rendering the iPS cells suitable for propagation in the infarcted heart.

To the best of Applicant's knowledge and the results reported in Example 2, below, that isoxazoles and other similar small molecules work on other stem cells from the body. For example, stem cells derived from the bone marrow and the heart can be directly reprogrammed into myoctyes or endothelial cells with small molecule drugs like isoxazoles. Thus, isoxazoles and similar small molecules can be directly administered, e.g., by injection into the hearts of patients, e.g., heart attack patients, for converting inflammatory cells and stem cells mobilized to the ischemic sites into cardiac cells in order to replace the scar tissue. The net effect is to assist with the regrowth of the damaged heart after the heart attack. The disclosed methods have the advantage of being non-viral based, using a previously approved compound, that simplifies clinical adoptions. Moreover, it is Applicant's belief that the disclosed methods will dramatically increase cardiac progenitors within a few short weeks after treating with isoxazoles or other similar small molecules like cardionogin; CDNG1/vuc230, CDNG2/vuc198, and CDNG3/vuc247.

Current approaches in using iPS cells include limitations such as genetic mutations and or tumor (i.e., cancer) growth versus Applicant's methods, approach and technique. The methods are beneficial in that genetic mutations are not necessary, improving safety for clinical use.

It is Applicant's belief and to the best of his knowledge, the current reported study provides the first evidence that iPS cells can be therapeutically rendered safe for use by altering their chromatin configuration for upregulation of cardiac genes. These so called cardiac progenitors can propagate and regenerate the dying myocardium with limited cell death. Chemical reprogramming iPS cells to desired cardiac lineage would be smart strategy in cardiac therapeutics.

### Experimental Methods

### Experiment No. 1

### Maintenance of mouse SiPS

SiPS were maintained on mitomycin C-treated mouse embryonic fibroblasts (MEFs) dishes in Knock out Dulbecco's Modified Eagle's Medium (knock out-DMEM, Invitrogen, CA, USA) supplemented with 20% Knockout Serum Replacement (KSR; Invitrogen, USA), 0.1 mM MEM Non-Essential Amino Acids solution (Invitrogen, CA, USA), 0.2 mM L-glutamine (Invitrogen, USA), 0.1 mM β- mercaptoethanol (Invitrogen, CA, USA) and 1 000 U/ml LIF (Millipore) 0.5% penicillin and streptomycin. The colonies thus generated were detached regularly at an interval of 3-4 days with 0.2% collagenase-1V (Invitrogen, CA, USA) dissociated into single cell suspension with 0.025% trypsin (Sigma Aldrich, MO, USA) and re-plated onto MEFs for propagation.

### Cell proliferation assay

The cell proliferation assay was performed with the use of the MTS [3-(4,5-dimethylthiazol-2-*yl*)-5-(3-carboxymethoxyphenyl)-2-(4-sulfophenyl)-2H-tetrazolium] assay according to the manufacturer's recommendations (Promega). The plates were read at 490 nm using an automated ELISA plate-reader for the quantity of formazan product which was directly proportional to the number of living cells in culture.

### DNA methyltransferase (DNMT) activity assay

Nuclear extracts were isolated using the NE-PER Nuclear and Cytoplasmic Extraction Kit (Thermo Scientific, IL USA). Total DNMT activity was determined using an EpiQuik DNA methyltransferase activity assay kit (Epigentek, Brooklyn, NY) per manufacturer's protocol. Enzyme activity for samples and controls was measured on a microplate reader (Hidex Chameleon, Finland) at 450 nm and DNMT activity (OD/h/ml) was calculated according to the formula: (Sample OD-blank OD)/(sample volume)×1000.

### RT-PCR and Quantitative RT-PCR

Total RNA from small molecule treated and non-treated SiPS cells was isolated using RNeasy mini kit (Qiagen, Maryland, USA) and Omniscript Reverse Transcription kit (Qiagen, Maryland, USA) was used for the respective cDNA synthesis per manufacturer's instructions. For PCR amplification, 1 µg of the cDNA from the reverse transcription reaction was added to PCR mix containing the suggested quantity of the PCR buffer, Q solution, dNTP mix, reverse and forward primers, Taq DNA polymerase and distilled water. PCR conditions included initial denaturation at 95°C for 4 minutes, 32 cycles of denaturation at 95°C for 1 minute, annealing at 55°C for 1 minute, extension at 72°C for 1 minute and final extension at 72°C for 7 minutes. The PCR products were separated on 1.5 % agarose gel, stained with ethidium bromide and visualized and photographed on a UV transluminator (Bio-Rad, USA).

### Myocardial Infarction Model

The animals were anesthetized with (ketamine/xylazine 0.05 ml intra-peritonealy). A midline cervical skin incision was performed for intubation. The animals were mechanically ventilated with room air supplemented with oxygen (1.5 L/min) using a rodent ventilator (Model 683, Harvard Apparatus, MA, USA). Body temperature was carefully monitored with a probe (Cole-Parmer Instrument, IL, USA) and was maintained at 37°C throughout the surgical procedure. The heart was exposed by left-sided limited thoracotomy and the left anterior descending (LAD) coronary artery was ligated with a prolene #9-0 suture. Myocardial ischemia was confirmed by color change of the left ventricular wall. The cells were injected 10 minutes after coronary artery ligation at multiple sites (3-4 sites per heart) in the free wall of the left ventricle under direct vision. For post-engraftment tracking of the transplanted cells and determination of their fate, the cells were labeled with PKH26 (Sigma, Product# PKH26-GL) according to manufacturer's instructions. The chest was closed and the animals were allowed to recover. To alleviate pain, Buprinex (0.05 ml) was injected subcutaneously in first 24 hours of surgery. The animals were euthanized on 7 days 4 weeks and 6-8 weeks after transthoracic echocardiography for the heart function evaluation. The hearts were frozen or fixed with 10% formalin solution and processed for embedding in paraffin for immunohistological studies.

### Immunocytochemistry

For immunocytochemistryr differentiated colonies of SiPS were immunostained with respective specific primary antibodies (anti-Oct3/4, anti-Sox2, anti Nanog antibodies, all at 1:100 concentration; Cell Signaling, Danvers, USA). Small molecule treated SIPs were seeded on 0.1 % gelatin coated chambered slides for immunostaining. The cells were fixed with PBS containing 4% paraformaldehyde for 10 minutes at room temperature. After washing with PBS, the cells were blocked for 45 minutes at room temperature by CAS block (Invitrogen, CA, USA) and were immunostained with antigen specific primary antibodies Nkx2.5,α Sarcomeric actin.(Santa Cruz, CA, USA). The primary antibody-antigen reaction was detected with fluorescently conjugated specific secondary antibodies. Nuclei were stained with 5 µg/ml 4'6-diamidino-2- phenyl indole (DAPI; Invitrogen, CA, USA) staining. Fluorescence signals were observed and photographed using fluorescence microscopy (Olympus; Tokyo, Japan).

### Gene Expression Profiling

Affymetrix array-based gene expression profiling further confirmed 2-3 folds downregulation of Dnmt1, Dnmt3b and Max gene associated protein which were associated with global DNA hypomethylation and myc dependent cell transformation. In addition, there was 2-3 folds concomitant upregulation of CCL7, CXCR2, CXCR5, integral membrane protein 2A, and EphrinA3.

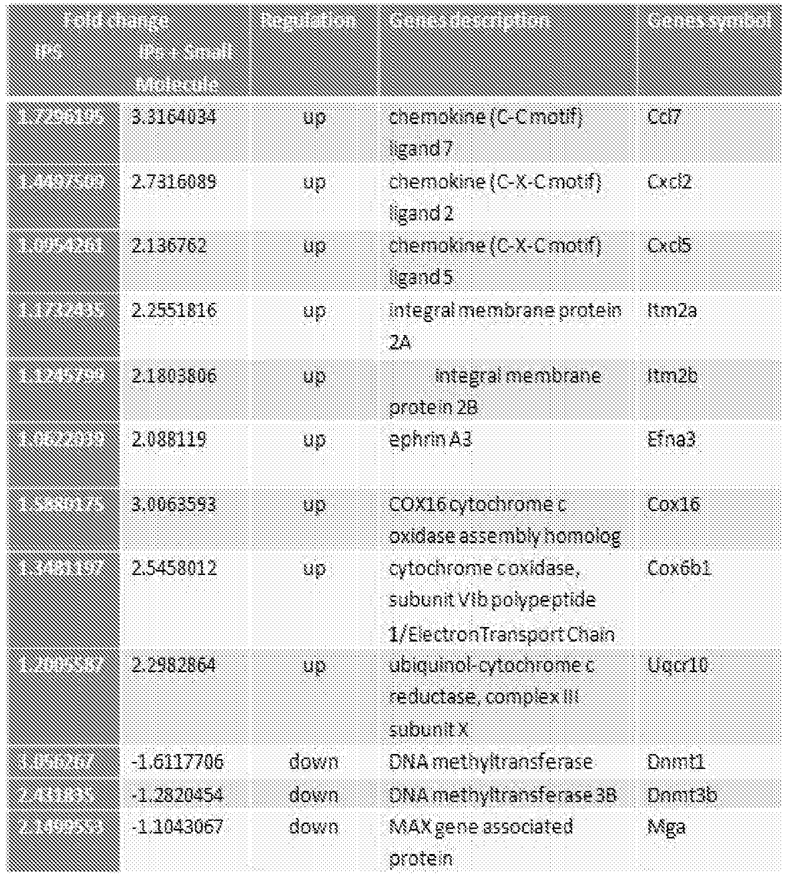

### Experiment No. 2

Applicant has identified a bone marrow stem cell population named small juvenile stem cells (SJSCs) from aged mouse which express pluripotency and cardiac markers. Applicant expects when these cells are treated with isoxazole compound, would be more appropriate for cardiac differentiation and can be used as an alternate to IPS cells (8).

### Experiment No. 3

This experiment discloses an alternate method of generating cardiac progenitors by preconditioning with electrical stimulation supplemented with cardiogenic small molecules. Applicant identified another cell population (9) that expresses hematopoietic progenitor marker (c-kit), pluripotency markers (Oct-4, Sox2, Nanog), a stem cell side population marker (Bcrp1), early cardiac lineage markers (Nkx2.5, GATA4, MEF2C), and a vascular progenitor marker (Flk1). Pre-conditioning (PC) of stem cells either through a brief period of ischaemia/anoxia or treatment with alternative mimetic improves their post-engraftment survival and differentiation characteristics. However there is no known report regarding the role of PC with electric stimulation (EleS) in stem cell survival, adhesion and cardiac differentiation. The heart generates a constant electrical field, the effect of which has not been explored in stem cells prior to transplantation. This study demonstrated that EleS provided PC effects on the survival of cardiac stem cells (Sca-1⁺ CSCs) through an increase in cell adhesion via focal adhesion kinase (FAK) activation, and releasing connective tissue growth factor (CTGF) by miR-378 down-regulation. It was found that connective tissue growth factor (*Ctgf*) was responsible for EleS-induced CSC(^{EleS}CSCs) survival and adhesion. Importantly, knockdown of *Ctgf* abolished EleS-induced cytoprotective effects and recovery of cardiac function. Furthermore, miR-378 was identified as a potential *Ctgf* regulator in ^{EleS}CSCs. This is another stem cell type which expresses both pluripotency and cardiac genes,and these cells can be further exploited with isoxazoles for cardiac progenitors.

### Isolation of Sca-1⁺ CSCs

C57BL6 mice (Harlan) were used for isolation of CSCs. 12 weeks old C57BL6 mice were anesthetized by intraperitoneal injection of ketamine/xylazine (87-100 mg and 13-15 mg/kg, respectively). The depth of anesthesia was monitored by positive toe pinch and muscle relaxation. Hearts were extracted and washed with ice-cold PBS to remove blood cells. After removal of aorta, pulmonary artery, and pericardium, the whole hearts were minced and digested for 20 min at 37°C with 0.1% type-II collagenase (Invitrogen) and 0.01% DNase I (Worthington Biochemical Corporation). The cells obtained were passed through 40 µm filter to remove the debris, fractionated with 70% Percoll (Fluka) and cultured in maintenance medium containing serum-free DMEM/F12 (Invitrogen) supplemented with B27 (Invitrogen), 20 ng/ml EGF (Sigma), and 40 ng/ml bFGF (basic fibroblast growth factor, Peprotech). One week later, the cells were transferred to new dishes with serum-free maintenance medium with a density of 100 cells/cm² to initiate colony formation and each colony was mechanically picked up for individual sub-culture in 24 well dishes in DMEM/F12 (Invitrogen) supplemented with 2% FBS, B27 supplement, 20 ng/ml EGF, 40 ng/ml bFGF, and 10 ng/ml LIF (Leukemia inhibitory factor, Millipore). Colony-derived cells were re-seeded on new dishes at 90% confluence and were maintained with DMEM/F12 with 2% FBS.

### EleS of CSCs

Twenty four hours after seeding at a cell density of 3 × 10⁵ cells/35 mm dish, the cells were serum-starved for 15 hr followed by EleS (^{EleS}CSCs) using a culture cell pacer system (lonOptix). Cells were subjected to EleS for 0, 1, and 3 hr at 1.5 V/1.8 cm with biphasic square pulse (5 ms) at 5 Hz frequency. Cells without EleS (^{Non-EleS}CSC) were used as baseline controls. The cells were later harvested and used for various molecular and cellular studies.

Unless otherwise defined, all technical and scientific terms used herein have the same meaning as commonly understood by one of ordinary skill in the art to which this invention belongs. All nucleotide sequences provided herein are presented in the 5' to 3' direction.

The inventions illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed.

### References

1. Shi Y, Do JT, Desponts C, Hahm HS, Schöler HR, Ding S (2008). A combined chemical and genetic approach for the generation of induced pluripotent stem cells. Cell Stem Cell. Jun 5;2(6):525-8.
2. Huangfu D, Maehr R, Guo W, Eijkelenboom A, Snitow M, Chen AE, Melton DA (2008). Induction of pluripotent stem cells by defined factors is greatly improved by small-molecule compounds. Nat Biotechnol. Jul;26(7):795-7.
3. Zhu S, Li W, Zhou H, Wei W, Ambasudhan R, Lin T, Kim J, Zhang K, Ding S (2010). Reprogramming of human primary somatic cells by OCT4 and chemical compounds.Cell Stem Cell, vol. 7, no. 6, pp. 651-655.
4. C. Desponts and S. Ding (2010). Using small molecules to improve generation of induced pluripotent stem cells from somatic cells. Methods in Molecular Biology, vol. 636, pp. 207-218.
5. Pasha Z, Haider HKh, Ashraf M (2011). Efficient non-viral reprogramming of myoblasts to sternness with a single small molecule to generate cardiac progenitor cells. PLoS One.;6(8):e23667.
6. Sadek H, Hannack B, Choe E, Wang J, Latif S, Garry MG, Garry DJ, Longgood J, Frantz DE, Olson EN, Hsieh J, Schneider JW (2008). Cardiogenic small molecules that enhance myocardial repair by stem cells.Proc Natl Acad Sci USA. Apr 22;105(16):6063-8.
7. Russell JL, Goetsch SC, Aguilar HR, Frantz DE, Schneider JW. Targeting native adult heart progenitors with cardiogenic small molecules. ACS Chem Biol. 2012 Jun 15;7(6):1067-76.
8. Igura K, Okada M, Kim HW, Ashraf M. Identification of small juvenile stem cells in aged bone marrow and their therapeutic potential for repair of the ischemic heart. Am J Physiol Heart Circ Physiol. 2013 Nov 1;305(9):H1354-62.
9. Kim SW, Kim HW, Huang W, Okada M, Welge JA, Wang Y, Ashraf M.Cardiac stem cells with electrical stimulation improve ischaemic heart function through regulation of connective tissue growth factor and miR-378. Cardiovasc Res. 2013 Nov 1;100(2):241-51.

## Claims

1. An *in vitro* method for preparing a cardiac progenitor cell or a cardiomyocyte from an induced pluripotent stem (iPS) cell, comprising contacting the stem cell with an effective amount of about 0.5 µM to about 30 µM of an isoxazole to prepare the cardiac progenitor cell or the cardiomyocyte from the induced pluripotent stem cell.

2. The method of claim 1, wherein the cardiac cell overexpresses one or more cardiac genes or markers such as Nkx 2.5, GATA4, αMHC, Sarcomeric actin, Gai, mir-133, mir-762, CCL7, CXCR2, CXC5, integral membrane protein 2A, or ephrin A3, wherein the induced pluripotent stem cell preferably expresses cell surface markers CD29(+), CD44(+), CD59(+), CD90(+).

3. The method of claim 1, wherein the cardiac cell under expresses one or more pluripotent genes or markers such as one or more of miR-290-295 cluster, let-7 family, Dnmt1, Dnmt3b, and Max.

4. The method of claim 1, wherein the iPS cell was created by a method comprising contacting a parent cell with an effective amount of a DNA methyltransferase inhibitor to upregulate Oct4, wherein the iPS cell was preferably created by a method that excludes the insertion of exogenous genes into the parent cell.

5. The method of claim 1, wherein the iPS stem cell expresses cell surface marker c-Kit and/or FIk1.

6. An *in vitro* method for preparing a chemically modified induced pluripotent stem (iPS) cell **characterized by** DNA hypomethylation, wherein the iPS cell is modified by contacting the iPS cell with an effective amount of about 0.5 µM to about 30 µM of an isoxazole.

7. A method for preparing a substantially pure cardiac progenitor and/or cardiomyocyte population, **characterized in that** the cardiac progenitor and/or cardiomyocyte population is derived from an iPS cell prepared by means of the method according to claim 6.

8. A method according to claim 7, wherein the population is substantially homogenous.

9. A method for preparing a clonal population of cells prepared by means of the method according to claim 7, preferably comprising a pharmaceutically acceptable carrier for therapeutic use.

## Patentansprüche

1. *In vitro*-Verfahren zur Herstellung einer kardialen Vorläuferzelle oder eines Kardiomyozyten aus einer induzierten pluripotenten Stammzelle (iPS), umfassend das Inkontaktbringen der Stammzelle mit einer wirksamen Menge von etwa 0,5 µM bis etwa 30 µM eines Isoxazols, um die kardiale Vorläuferzelle oder den Kardiomyozyten aus der induzierten pluripotenten Stammzelle herzustellen.

2. Verfahren nach Anspruch 1, wobei die Herzzelle ein oder mehrere Herzgene oder-marker wie Nkx 2.5, GATA4, αMHC, sarkomeres Aktin, Gαi, mir-133, mir-762, CCL7, CXCR2, CXC5, integrales Membranprotein 2A oder Ephrin A3 überexprimiert, wobei die induzierte pluripotente Stammzelle vorzugsweise die Zelloberflächenmarker CD29(+), CD44(+), CD59(+), CD90(+) exprimiert.

3. Verfahren nach Anspruch 1, wobei die Herzzelle ein oder mehrere pluripotente Gene oder Marker unterexprimiert, wie z.B. einen oder mehrere der Cluster miR-290-295, let-7-Familie, Dnmt1, Dnmt3b und Max.

4. Verfahren nach Anspruch 1, wobei die iPS-Zelle durch ein Verfahren erzeugt wurde, das das Inkontaktbringen einer Mutterzelle mit einer wirksamen Menge eines DNA-Methyltransferase-Inhibitors zur Hochregulierung von Oct4 umfasst, wobei die iPS-Zelle vorzugsweise durch ein Verfahren erzeugt wurde, das die Einfügung exogener Gene in die Mutterzelle ausschließt.

5. Verfahren nach Anspruch 1, wobei die iPS-Stammzelle den Zelloberflächenmarker c-Kit und/oder Flk1 exprimiert.

6. *In vitro*-Verfahren zur Herstellung einer chemisch modifizierten induzierten pluripotenten Stammzelle (iPS-Zelle), die durch DNA-Hypomethylierung gekennzeichnet ist, wobei die iPS-Zelle durch Inkontaktbringen der iPS-Zelle mit einer wirksamen Menge von etwa 0,5 µM bis etwa 30 µM eines Isoxazols modifiziert wird.

7. Verfahren zur Herstellung einer im Wesentlichen reinen kardialen Vorläufer- und/oder Kardiomyozytenpopulation, **dadurch gekennzeichnet, dass** die kardiale Vorläufer- und/oder Kardiomyozytenpopulation von einer iPS-Zelle abgeleitet ist, die mit Hilfe des Verfahrens nach Anspruch 6 hergestellt wurde.

8. Verfahren nach Anspruch 7, wobei die Population im Wesentlichen homogen ist.

9. Verfahren zur Herstellung einer klonalen Population von Zellen, die mittels des Verfahrens nach Anspruch 7 hergestellt wurden, vorzugsweise umfassend einen pharmazeutisch akzeptablen Träger zur therapeutischen Verwendung.

## Revendications

1. Méthode *in vitro* de préparation d'une cellule progénitrice cardiaque ou d'un cardiomyocyte à partir d'une cellule souche pluripotente induite (iPS), comprenant la mise en contact de la cellule souche avec une quantité efficace d'environ 0,5 µM à environ 30 µM d'un isoxazole pour préparer la cellule progénitrice cardiaque ou le cardiomyocyte à partir de la cellule souche pluripotente induite.

2. La méthode de la revendication 1, dans laquelle la cellule cardiaque surexprime un ou plusieurs gènes ou marqueurs cardiaques tels que Nkx 2.5, GATA4, αMHC, actine sarcomérique, Gαi, mir-133, mir-762, CCL7, CXCR2, CXC5, protéine membranaire intégrale 2A ou éphrine A3, la cellule souche pluripotente induite exprimant de préférence les marqueurs de surface cellulaire CD29(+), CD44(+), CD59(+), CD90(+).

3. Méthode de la revendication 1, dans laquelle la cellule cardiaque sous-exprime un ou plusieurs gènes ou marqueurs pluripotents tels qu'un ou plusieurs des groupes miR-290-295, la famille let-7, Dnmt1, Dnmt3b et Max.

4. La méthode de la revendication 1, dans laquelle la cellule iPS a été créée par une méthode comprenant la mise en contact d'une cellule parentale avec une quantité efficace d'un inhibiteur d'ADN méthyltransférase pour surréguler Oct4, la cellule iPS étant de préférence créée par une méthode qui exclut l'insertion de gènes exogènes dans la cellule parentale.

5. La méthode de la revendication 1, dans laquelle la cellule souche iPS exprime le marqueur de surface cellulaire c-Kit et/ou Flk1.

6. Méthode *in vitro* de préparation d'une cellule souche pluripotente induite (iPS) chimiquement modifiée, **caractérisée par** une hypométhylation de l'ADN, dans laquelle la cellule iPS est modifiée par contact avec une quantité efficace d'environ 0,5 µM à environ 30 µM d'un isoxazole.

7. Méthode de préparation d'une population de progéniteurs cardiaques et/ou de cardiomyocytes sensiblement pure, **caractérisée en ce que** la population de progéniteurs cardiaques et/ou de cardiomyocytes est dérivée d'une cellule iPS préparée au moyen de la méthode selon la revendication 6.

8. Méthode selon la revendication 7, dans laquelle la population est sensiblement homogène.

9. Méthode de préparation d'une population clonale de cellules préparées au moyen de la méthode selon la revendication 7, comprenant de préférence un support pharmaceutiquement acceptable pour un usage thérapeutique.
